(19) Europäisches Patentamt
European Patent Office
Office européen des brevets

(11) **EP 3 402 502 B1**

(12) **FASCICULE DE BREVET EUROPEEN**

(45) Date de publication et mention de la délivrance du brevet:
**22.10.2025 Bulletin 2025/43**

(21) Numéro de dépôt: **17711706.6**

(22) Date de dépôt: **12.01.2017**

(51) Classification Internationale des Brevets (IPC):
**A61K 36/8968** (2006.01)     **A61P 17/00** (2006.01)
**A61K 31/702** (2006.01)

(52) Classification Coopérative des Brevets (CPC):
**A61K 36/8968; A61K 31/702; A61P 17/00**

(86) Numéro de dépôt international:
**PCT/FR2017/050070**

(87) Numéro de publication internationale:
**WO 2017/121965 (20.07.2017 Gazette 2017/29)**

(54) **PRINCIPE ACTIF OBTENU A PARTIR D'UN HYDROLYSAT D'OPHIOPOGON JAPONICUS POUR LE TRAITEMENT DE LA DERMATITE ATOPIQUE**

WIRKSTOFF AUS DEM HYDROLYSAT OPHIOPOGON JAPONICUS ZUR BEHANDLUNG VON DER ATOPISCHEN DERMATITIS

ACTIVE INGREDIENT OBTAINED FROM A HYDROLYSAT OF OPHIOPOGON JAPONICUS FOR USE IN THE TREATMENT OF ATOPIC DERMATITIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.01.2016 FR 1650228**

(43) Date de publication de la demande:
**21.11.2018 Bulletin 2018/47**

(73) Titulaire: **Societe Industrielle Limousine d'Application Biologique**
**19240 Saint Viance (FR)**

(72) Inventeur: **PAUFIQUE, Jean**
**19130 Objat (FR)**

(74) Mandataire: **Aquinov**
**12, Cours Xavier Arnozan**
**33000 Bordeaux (FR)**

(56) Documents cités:
**WO-A1-02/47704    WO-A1-2014/081715**
**FR-A1- 2 930 729**

- **DATABASE WPI Week 201437, Derwent World Patents Index; AN 2014-J43671, XP002761998**
- **DATABASE WPI Week 201432, Derwent World Patents Index; AN 2014-E98201, XP002761999**
- **DATABASE WPI Week 201230, Derwent World Patents Index; AN 2012-E60748, XP002762000**
- **DATABASE WPI Week 201126, Derwent World Patents Index; AN 2011-A47481, XP002762001**
- **DATABASE WPI Week 201424, Derwent World Patents Index; AN 2014-E98228, XP002762002**
- **DATABASE WPI Week 200548, Derwent World Patents Index; AN 2005-474609, XP002762003**
- **DATABASE WPI Week 201439, Derwent World Patents Index; AN 2014-L47189, XP002762004**
- **DATABASE WPI Week 201059, Derwent World Patents Index; AN 2010-J98069, XP002762005**
- **ANDO Y ET AL: "Skin-moisture-retaining agent for cosmetics - consists of sugars extracted from ophiopogon and liriope genus plants", WPI/ THOMSON,, vol. 1987, no. 20, 14 April 1987 (1987-04-14), XP002511306**
- **TOSHIAKI MAKINO ET AL: "Effect of Bakumijiogan, an Herbal Formula in Traditional Chinese Medicine, on Atopic Dermatitis-Like Skin Lesions Induced by Mite Antigen in NC/Jic Mice", BIOL. PHARM. BULL., 3 September 2008 (2008-09-03), pages 2108 - 2113, XP055368121, Retrieved from the Internet <URL:https://www. jstage.jst.go.jp/article/bpb/31/11/31_11_2108/ _pdf> [retrieved on 20170427]**

**Description**

La présente invention se rapporte au traitement de la dermatite atopique.

La dermatite atopique, aussi appelé eczéma atopique, est une maladie chronique de la peau d'origine génétique et environnementale. Elle atteint aussi bien l'homme que les animaux principalement les chiens et les chats.

Chez l'homme, la dermatite atopique est la dermatose la plus fréquente chez les enfants, et peut apparaître dès les premiers mois de la vie. Elle atteint 20 % des enfants de moins de 7 ans, se situe encore autour de 18 % chez les enfants de 7 à 16 ans, et 3% des adultes sont toujours atteints. La notion de chronicité et d'évolution à rechutes est importante.

La prévalence (nombre de patients atteints dans la population générale) de la dermatite atopique a triplé en 30 ans dans les pays industrialisés. Cette prévalence s'accroît en particulier avec l'environnement, les modifications du mode de vie et l'augmentation de l'hygiène dans nos sociétés industrialisées. Il s'agit donc un problème émergent de santé publique.

Chez l'animal, la dermatite atopique est une dermatose inflammatoire chronique et prurigineuse d'origine multifactorielle. Cette maladie résulte d'interactions complexes entre les facteurs de prédisposition génétique et des facteurs environnementaux. Les premiers signes cliniques de la maladie chez les chiens ou les chats apparaissent généralement entre 6 mois et 3 ans. Les lésions impliquent principalement la face, les extrémités, les grands plis et l'abdomen. Il est estimé qu'elle touche environ 10 à 15% des populations canines et félines. Les animaux étant soumis aux mêmes agressions environnementales que leurs maîtres, la prévalence de la dermatite atopique tend à s'accroître chez le chien comme chez l'Homme, faisant de cette maladie une préoccupation de premier ordre pour les vétérinaires.

Chez l'homme, quel que soit l'âge, l'aspect de la dermatite atopique est identique, avec une sécheresse cutanée par manque de film hydrolipidique protecteur et des plaques d'eczéma, rouges, prurigineuses, épaissies (lichenifiées) avec éventuellement suintement.

Il s'agit d'une maladie multifactorielle complexe qui rend son traitement difficile. Tous les symptômes de la pathogénèse de la dermatite atopique (peau sèche, les tâches rouges, les démangeaisons, des éruptions cutanées et des gonflements, dans les périodes de crise et d'accalmie) sont associés principalement à la destruction de la barrière cutanée sur des zones lésées, mais aussi des zones non lésées, dû à la faible synthèse des protéines de différenciation comme la cytokeratine 10, l'involucrine, laloricrine et la filaggrine.

Les symptômes de la dermatite atopique sont aussi caractérisés par une sur-expression des molécules pro-inflammatoires comme les interleukines (IL-) 4, 8, 13 et le thymic stromal lymphopoietin (TSLP). Dans ce cercle vicieux, la réponse inflammatoire impacte sur la synthèse de filaggrine. En présence de cytokines Th2, les kératinocytes différenciés réduisent significativement l'expression du gène FLG (filaggrine). La réponse inflammatoire atopique est le facteur central augmentant la destruction de la fonction barrière cutanée.

Deux groupes de gènes impliqués dans la dermatite atopique ont été identifiés : des gènes codant pour le système immunitaire et des gènes codant pour des protéines de structure épidermique.

La dermatite atopique des canins et des félins partage de nombreuses similitudes cliniques et immunologiques avec la dermatite atopique humaine. Elle est associée à une altération de la barrière cutanée et dans la plupart des cas à la production d'anticorps IgE dirigés contre des allergènes environnementaux (Marsella and Girolomoni « Canine models of atopic dermatitis: a useful tool with untapped potential », J Invest Dermatol, 2009 Oct;129(10):2351-7; Marsella et al «Current evidence of skin barrier dysfunction in human and canine atopic dermatitis » Vetirinary Dermatology, 22, 239-248, 2011 ; Bizikowa et al « Review : Clinical and histological manifestations of canine atopic dermatitis », Vet Dermatol 2015 ; 26 : 79-e24 ; ; Bizikowa et al « Review : Role of genetics and the environement in the pathogenesis of canine atopic dermatitis », Vet Dermatol 2015 ; 26 : 95-e26).

En utilisant un modèle expérimental canin atopique, la surexpression de cytokines reflétant un biais TH2 au niveau des zones lésées a été mise en évidence, de façon similaire à celui observé chez l'humain (Olivry et al, « Early Activation of Th2/Th22 Inflammatory and Pruritogenic Pathways in Acute Canine Atopic Dermatitis Skin Lesions » Journal of Investicative Dermatology, 2016).

La majorité des produits existants destinés à traiter les peaux atopiques, chez l'homme ou l'animal, agissent sur un seul des facteurs de la maladie :

- soit en limitant l'inflammation : il s'agit essentiellement de dermocorticoïdes (par exemple, la dexaméthasone, corticoïde synthétique) qui calme temporairement le cercle de l'inflammation,
- soit en améliorant la barrière cutanée : il s'agit notamment d'émollients qui colmatent la barrière cutanée altérée afin de bloquer mécaniquement l'hydratation dans la peau.

Il existe aussi des ingrédients issus de plantes, mais ceux-ci agissent essentiellement comme anti-inflammatoires ou comme agents restructurant de la barrière cutanée.

L'objectif de la présente invention est de proposer un principe actif obtenu à partir d'une plante capable d'agir sur différents facteurs de la dermatite atopique humaine ou animale. A cet effet, l'invention vise un principe actif obtenu à partir d'un hydrolysat *d'Ophiopogon japonicus* pour une utilisation, en application topique sur la peau humaine ou animale, dans le traitement de la dermatite atopique.

*L'Ophiopogon japonicus* est une espèce herbacée vivace, basse et foisonnante, à rhizome de la famille des Liliacées.

Elle est cultivée comme plante ornementale couvrante à rhizome tubéreux présentant de très nombreuses utilisations en médecine traditionnelle chinoise.

L'espèce est originaire du Japon et de Corée, et est également cultivée au Vietnam et en Chine, notamment dans les provinces du Sichuan, Zhejiang et Hubei. Elle est appelée Muguet du Japon en France, et Mondo grass, Fountain plant, Monkey grass ou Dwarf lilyturf en anglais. La racine tubéreuse a une longueur de quelques centimètres ; elle est de couleur jaune clair à jaune-brun à l'extérieur, avec des rides longitudinales. Son odeur est faible, et son goût légèrement sucré et mucilagineux.

En médecine traditionnelle chinoise, la racine est connue pour « nourrir les Poumons et le Yin, pour nourrir l'Estomac et produire des Fluides, pour éloigner la Chaleur du cœur et pour apaiser l'Esprit ». Les principales indications thérapeutiques concernent les toux sèches, les maux de gorge, l'insomnie, l'irritabilité, la constipation et la diphtérie, selon la 16ème édition de la Pharmacopée Japonaise.

Bien que des compositions topiques comprenant un mélange de plusieurs plantes dont *Ophiopogon japonicus* soient connues, notamment dans la médecine traditionnelle (par exemple dans les documents suivants, WO2014081715, CN103656323, CN103520572, CN102406570, CN101904985, CN103520475, KR20050014947, WO0247704, CN103736068, CN101757419 Ando Y et al., Toshiaki Makino et al.), aucun enseignement ne divulgue un extrait *d'Ophiopogon japonicus* seul pour traiter la dermatite atopique.

Les racines *d'Ophiopogon japonicus* sont issues de filières cultivées de Chine et de Vietnam.

Des extraits *d'Ophiopogon japonicus* sont connus en cosmétique, en particulier dans le brevet FR2930729 comme hydratant par action sur le taux de NMF et sur la formation des jonctions serrées des couches. Mais les ingrédients hydratants ne sont pas obligatoirement efficaces sur les différents marqueurs de la dermatite atopique, et de façon surprenante, selon l'invention, un principe actif obtenu à partir d'un hydrolysat *d'Ophiopogon japonicus* permet de traiter les multiples facteurs de la dermatite atopique. Il diminue le taux de rechute, la gravité, l'intensité et la fréquence des poussées des crises de la dermatite atopique, il diminue l'importance de l'eczéma et améliore la qualité de vie des patients et des familles ou la qualité de vie des animaux et de leurs propriétaires.

Son mode d'action repose à la fois sur l'inflammation (les marqueurs de l'inflammation et les gènes liés à l'inflammation), sur la fonction barrière cutanée (les marqueurs de cohésion épidermique, les marqueurs de la différenciation et les gènes associés à la différenciation) et sur l'organisation et la conformation des lipides épidermiques. Il permet ainsi de consolider la construction épidermique et l'intégrité et la résistance de la barrière cutanée, afin de limiter l'adhésion des bactéries sur la peau.

D'autres caractéristiques et avantages ressortiront de la description en détail de l'invention qui va suivre, en regard des figures annexées :

- La Figure 1A représente l'image visualisée sur microscope d'une coupe d'épiderme humain reconstruit normal (correspondant au résultat du Tableau 15 - REh normal - Témoin, morphologie générale +++),
- La Figure 1B représente l'image visualisée sur microscope d'une coupe d'épiderme humain reconstruit inflammé (correspondant au résultat du Tableau 15 - REhI - Témoin, morphologie générale -),
- La Figure 1C représente l'image visualisée sur microscope d'une coupe d'épiderme humain reconstruit inflammé traité par voie topique avec un principe actif selon l'invention (correspondant au résultat du Tableau 15 - REhI - Exemple 1 à 1%, morphologie générale ++),
- La Figure 1D représente l'image visualisée sur microscope d'une coupe d'épiderme humain reconstruit inflammé présentant une barrière altérée (correspondant au résultat du Tableau 15 - REhBAI - Témoin, morphologie générale -),
- La Figure 1E représente l'image visualisée sur microscope d'une coupe d'épiderme humain reconstruit inflammé présentant une barrière altérée traité par voie topique avec un principe actif selon l'invention (correspondant au résultat du Tableau 15 - REhBAI - Exemple 1 à 1%, morphologie générale ++),
- La Figure 2A représente l'image visualisée au microscope d'une coupe d'épiderme humain reconstruit normal marquée avec une sonde fluorescente (le lucifer yellow) (correspondant au résultat du Tableau 16 - REh normal - Témoin, mesure de pénétration du lucifer yellow : 4),
- La Figure 2B représente l'image visualisée au microscope d'une coupe d'épiderme humain reconstruit inflammé présentant une barrière altérée marquée avec une sonde fluorescente (le lucifer yellow) (correspondant au résultat du Tableau 16 - REhBAI - Témoin, mesure de pénétration du lucifer yellow : 85),
- La Figure 2C représente l'image visualisée au microscope d'une coupe d'épiderme humain reconstruit inflammé présentant une barrière altérée traité par voie topique avec un principe actif selon l'invention, marquée avec une sonde fluorescente (le lucifer yellow) (correspondant au résultat du Tableau 16 - REhBAI - Exemple 1 à 1%, mesure de pénétration du lucifer yellow : 51),
- La Figure 3A représente l'image du biofilm de *Staphylococcus aureus* formé sur l'épiderme reconstruit témoin soumis à une agression bactérienne,
- La Figure 3B représente l'image du biofilm de *Staphylococcus aureus* formé sur l'épiderme reconstruit traité par le principe actif selon l'invention soumis à une agression bactérienne.

DEFINITIONS

Par « principe actif » ou « actif « ou « extrait » au sens de l'invention, on entend au moins une molécule, préférentiellement un ensemble de molécules présentant un effet sur les cellules de la peau.

Par « principe actif obtenu à partir d'*Ophiopogon japonicus* » au sens de l'invention on entend toute molécule ou mélange de molécules obtenue(s) à partir d'*Ophiopogon japonicus.* Il peut s'agir de molécules natives de la plante ou de molécules obtenues par tout type de transformation des molécules natives de la plante, par exemple par hydrolyse. Le principe actif selon l'invention est un hydrolysat.

Par « hydrolysat » on entend tout extrait issu d'*Ophiopogon japonicus,* obtenu avec un procédé comprenant au moins une étape d'hydrolyse enzymatique ou chimique d'*Ophiopogon japonicus,* préférentiellement au moins une étape d'hydrolyse enzymatique. Par « *Ophiopogon japonicus* » on entend tout ou partie de la plante. Il peut s'agir de la plante entière ou d'une partie de la plante. Préférentiellement il s'agit de tubercules d'*Ophiopogon japonicus.*

Par « oligosaccharides » on entend des oligomères formés d'un nombre de monosaccharides par liaison glycosidique, le nombre d'unités de monosaccharides étant inférieur à 25 unités. Par « polysaccharides » on entend des polymères constitués de plusieurs oses liés entre eux par des liaisons osidiques, le nombre d'unités de monosaccharides étant supérieur à 25 unités. Par « peau atopique » on entend la peau d'une personne ou d'un animal souffrant de dermatite atopique.

DESCRIPTION DETAILLEE DE L'INVENTION

L'invention vise donc un principe actif obtenu à partir d'un hydrolysat d'*Ophiopogon japonicus* pour une utilisation dans le traitement de la dermatite atopique chez l'être humain ou chez l'animal. Le principe actif est appliqué en topique sur la peau de l'Homme ou de l'animal pour agir sur tous les paramètres de dermatite atopique. Il est utilisé sur les peaux atopiques pour :

- diminuer le taux de rechute,
- diminuer le score de gravité de la dermatite atopique (SCORAD),
- réduire l'intensité et la fréquence des poussées,
- diminuer l'importance de l'eczéma,
- améliorer l'index de qualité de vie des patients et des familles ou améliorer la qualité de vie de l'animal et des propriétaires.

Ces effets ont été évalués par des dermatologues en particulier sur un panel d'enfants à peaux atopiques.

Le principe actif selon l'invention agit sur :

- la fonction barrière cutanée, et/ou
- l'inflammation cutanée, et/ou
- le microbiote cutanée,

préférentiellement sur ces trois facteurs.

Ces efficacités sur les facteurs de la dermatite atopique sont expliquées puisque le principe actif selon l'invention agit en :

- diminuant l'inflammation des cellules cutanées, et/ou
- régularisant la cohésion des cellules cutanées, et/ou
- régularisant la différenciation des cellules cutanées, et/ou
- favorisant l'organisation et la conformation des lipides épidermiques, et/ou
- favorisant la construction épidermique, et/ou
- restaurant l'intégrité et la résistance de la fonction barrière de la peau, et/ou
- limitant l'adhésion des bactéries sur la peau.

Préférentiellement, il présente l'ensemble de ces effets.

En effet, le principe actif selon l'invention est capable d'agir notamment :

- sur les marqueurs de l'inflammation des cellules cutanées : en particulier il diminue la teneur en TSLP et en IL-8 dans ces cellules, et/ou
- sur les gènes associés à l'inflammation des cellules cutanées : en particulier il normalise l'expression du gène NELL2 et du gène Tenascine C dans ces cellules, et/ou
- sur les marqueurs de la cohésion épidermique : en particulier il augmente la claudine-1, et/ou
- sur les marqueurs de différenciation des cellules cutanées : en particulier il augmente la filaggrine, l'involucrine ou la loricrine dans ces cellules, et/ou
- sur les gènes associés à la différenciation des cellules cutanées : en particulier il stimule l'expression du gène FLG (filaggrine) et du gène LOR (Loricrine) et diminue l'expression du gène TGM1 (transglutaminase) dans ces cellules, et/ou
- Sur l'organisation et la conformation des lipides épidermiques, et/ou
- sur la construction morphologique de l'épiderme, et/ou
- sur l'intégrité et la résistance de la barrière épidermique, et/ou
- sur l'adhésion des bactéries sur la peau : en particulier il limite l'adhésion de *Staphylococcus aureus* sur la peau.

Préférentiellement, présente l'ensemble de ces effets.

Ces différents efficacités peuvent être démontrées sur tout modèle de peau atopique, en particulier sur un modèle cellulaire inflammé, sur les modèles d'épiderme humain reconstruit spécifiques tels dits REhI (épiderme reconstruit humain inflammé) et REhBAI (épiderme reconstruit humain inflammé présentant une barrière cutanée altérée) tels que décrits dans la publication P. Rouaud-Tinguely et al. « From the morphological to the transcriptomic characterization of a compromised three-dimensional in vitro model mimicking atopic dermatitis » British Journal of Dermatology (2015) 173, pp1006-1014 ou sur un modèle d'épiderme canin reconstruit spécifique tel dit REcl (épiderme reconstruit canin inflammé).

Le principe actif obtenu à partir d'un hydrolysat d'*Ophiopogon japonicus* utile selon l'invention dans le traitement de la dermatite atopique est préférentiellement un principe actif obtenu à partir d'*Ophiopogon japonicus* comprenant des sucres. Encore plus préférentiellement, il comprend des fructosanes, et plus particulièrement il comprend au moins 57% de fructosanes en poids par rapport au poids des sucres totaux du principe actif, encore plus préférentiellement au moins 80%. Les fructosanes sont des polysaccharides composés de fructose et de glucose.

Les sucres contenus dans le principe actif sont préférentiellement constitués par 45 à 80% de fructose, par 20 à 50% de glucose et par 0 à 5% de galactose. Ces sucres peuvent être sous forme de monomères, d'oligomères et de polymères. Majoritairement, les sucres contenus dans le principe actif sont des oligo et polysaccharides de poids moléculaires inférieurs à 400kDa sous forme de fructosanes. Ainsi, préférentiellement le principe actif selon l'invention comprend des oligo et polysaccharides de poids moléculaires inférieurs à 400kDa sous forme de fructosanes, représentant au moins 57% en poids des sucres présents dans le principe actif. Selon une variante particulièrement adaptée, le principe actif est obtenu à partir de tubercules d'*Ophiopogon japonicus.*

Selon un mode de réalisation, le principe actif utile selon l'invention se présente sous forme d'une poudre, en particulier une poudre de couleur claire, et présente au moins une des caractéristiques suivantes, préférentiellement toutes :

- un taux de matières sèches compris entre 900 et 1000mg/g,
- une teneur en sucres comprise entre 500 et 800mg/g, soit au moins 50% de sucres en poids par rapport au poids de matières sèches.

Le taux de matières sèches peut être mesuré par passage à l'étuve à 105°C d'un échantillon jusqu'à obtention d'un poids constant.

La teneur globale en sucres peut être déterminée par la méthode de DUBOIS sur une gamme de fructose (Dubois M. et al., Analytical chemistry, 28, 3, 350-356, 1956).

La caractérisation de la masse molaire des carbohydrates présents dans le principe actif de la présente invention peut être réalisée par méthode HPLC et le dosage des sucres simples par chromatographie liquide ionique.

Les masses molaires des carbohydrates sont évaluées par comparaison des temps de rétention des pics détectés dans les échantillons du principe actif avec les temps de rétention de standards injectés au préalable.

Les conditions opératoires sont préférentiellement les suivantes :

- Appareil : HPLC Agilent 1100 Series
- Colonnes : Colonnes PL aquagel-OH C60, C40, C30 avec pré-colonne de mêmes caractéristiques
- Mode d'élution : isocratique

- Phase mobile : Tampon NaNO$_3$ 0.3M + NaH$_2$PO4-2H$_2$O 0.01M pH7
- Longueurs d'onde de détection : UV 254nm et 280nm

Le principe actif selon l'invention est préférentiellement un principe actif obtenu en milieu aqueux à partir de tubercules d'*Ophiopogon japonicus.* Par « obtenu en milieu aqueux » on entend un milieu contenant principalement de l'eau, ou un milieu aqueux basique ou acide. Notamment il ne s'agit pas d'une huile, ni d'une huile essentielle.

Le principe actif selon l'invention est un hydrolysat d'*Ophiopogon japonicus,* préférentiellement un hydrolysat enzymatique.

Selon une variante particulièrement adaptée, le principe actif selon l'invention est un hydrolysat de tubercules d'*Ophiopogon japonicus,* préférentiellement un hydrolysat enzymatique de tubercules d'*Ophiopogon japonicus.*

En particulier, le principe actif utile selon l'invention, peut être obtenu par la mise en œuvre des étapes suivantes :

- solubilisation de poudre d'*Ophiopogon japonicus* (préférentiellement de tubercules) dans l'eau à raison d'au moins 50g/l,
- au moins une hydrolyse enzymatique des sucres,
- séparation des phases soluble et insoluble, par exemple par décantation,
- inactivation enzymatique par traitement thermique de la phase soluble.

L'inactivation enzymatique peut être suivie d'une ou plusieurs étapes de filtration(s) et/ou de concentration. Le principe actif peut être obtenu sous forme liquide ou sous forme de poudre par atomisation ou lyophilisation. Préférentiellement il est atomisé, en présence d'un adjuvant d'atomisation type maltodextrine, et utilisé sous forme de poudre.

Les paramètres des différentes étapes doivent être ajustés afin d'obtenir des principes actifs présentant les caractéristiques de l'invention, en particulier la présence de fructosanes présentant un poids moléculaire inférieur à 400 kDa.

Le principe actif selon l'invention est préférentiellement utilisé dans une composition dermatologique, cette composition comprenant un milieu dermatologiquement acceptable. Il s'agit de compositions dans différentes formes galéniques, adaptées à l'administration par voie topique cutanée.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Il s'agit préférentiellement d'une crème, d'un gel ou d'une pommade.

Il peut s'agir de compositions dermatologiques comprenant au moins 0,05% de principe actif obtenu à partir d'*Ophiopogon japonicus,* selon la présente invention, préférentiellement entre 0.05 et 1%.

Ces compositions comprennent, outre l'actif, un milieu physiologiquement acceptable et dermatologiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant dermatologiquement acceptable au moins un composé choisi parmi :

- les huiles, qui peuvent être choisies notamment parmi les huiles de silicone, linéaires ou cycliques, volatiles ou non volatiles,
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les épaississants et/ou gélifiants,
- les humectants, tels que les polyols comme la glycérine,
- les filtres organiques,
- les filtres inorganiques,
- les colorants, les conservateurs, les charges,
- les tenseurs,
- les séquestrants,
- et leurs mélanges, sans que cette liste soit limitative.

Des exemples de tels adjuvants sont cités notamment dans le Dictionnaire CTFA (*International Cosmetic Ingredient Dictionary and Handbook* publié par le *Personal Care Product Council*). Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Les compositions dermatologiques comprenant un principe actif selon l'invention peuvent donc être utilisées pour traiter la dermatite atopique chez l'être humain ou chez l'animal, en particulier chez le chien ou le chat.

Afin d'illustrer ces effets cosmétiques sur la peau, les exemples suivants avec leurs résultats d'essais sont présentés.

EXEMPLES

Exemple 1 : Principe actif selon l'invention

Le principe actif issu de tubercules d'*Ophiopogon japonicus* de l'exemple 1 est un hydrolysat caractérisé comme suit :

- Poudre de couleur beige claire,
- Matières sèches : 960mg/g
- Teneur en sucres :

    ◦ Monosaccharides : 8% par rapport à la matière sèche
    ◦ Oligo et polysaccharides : 88% par rapport à la matière sèche

- Teneur en cendres minérales : 2% par rapport à la matière sèche
- Teneur en protéines : 2% par rapport à la matière sèche

Exemple 2a : Composition dermatologique destinée à l'être humain se présentant sous forme de crème

La formule est composée d'une crème du commerce Diprobase® Cream de la société Merck dans laquelle a été ajouté le principe actif selon l'invention de l'exemple 1.

| Dénomination commerciale | Dénomination INCI | % incorporation MP ds produit | CAS n° |
|---|---|---|---|
| Diprobase® Cream | white soft paraffin | 99.95% | 8009-03-8 |
| | liquid paraffin | | 8012-95-1 |
| | cetostearyl alcohol | | - |
| | macrogol cetostearyl ether | | - |
| | Chlorocresol | | 59-50-7 |
| | sodium dihydrogen phosphate | | - |
| | sodium hydroxide | | - |
| | phosphoric acid | | - |
| | purified water | | 7732-18-5 |
| Principe actif selon l'invention | *Ophiopogon japonicus* root Extract | 0.05% | 952500-62-8 |

***Tableau 1***

Exemple 2b : Composition dermatologique destinée à l'être humain se présentant sous forme de crème

La formule est composée d'une crème du commerce Diprobase® Cream de la société Merck dans laquelle a été ajouté le principe actif selon l'invention de l'exemple 1.

| Dénomination commerciale | Dénomination INCI | % incorporation MP ds produit | CAS n° |
|---|---|---|---|
| | white soft paraffin | | 8009-03-8 |
| | liquid paraffin | | 8012-95-1 |
| | cetostearyl alcohol | | - |
| | macrogol cetostearyl ether | | - |
| Diprobase® Cream | Chlorocresol | 99.9% | 59-50-7 |
| | sodium dihydrogen phosphate | | - |
| | sodium hydroxide | | - |
| | phosphoric acid | | - |
| | purified water | | 7732-18-5 |
| Principe actif selon l'invention | *Ophiopogon japonicus* root Extract | 0.1% | 952500-62-8 |

**_Tableau 2_**

Exemple 2c : Composition dermatologique destinée à l'être humain se présentant sous forme de crème confort

La formule est composée d'une crème du commerce Diprobase® Cream de la société Merck dans laquelle a été ajouté le principe actif selon l'invention de l'exemple 1.

| Dénomination commerciale | Dénomination INCI | % incorporation MP ds produit | CAS n° |
|---|---|---|---|
| | white soft paraffin | | 8009-03-8 |
| | liquid paraffin | | 8012-95-1 |
| | cetostearyl alcohol | | - |
| | macrogol cetostearyl ether | | - |
| Diprobase® Cream | Chlorocresol | 99.7% | 59-50-7 |
| | sodium dihydrogen phosphate | | - |
| | sodium hydroxide | | - |
| | phosphoric acid | | - |
| | purified water | | 7732-18-5 |
| Principe actif selon l'invention | *Ophiopogon japonicus* root Extract | 0.3% | 952500-62-8 |

**_Tableau 3_**

Exemple 3 : Composition dermatologique destinée au chien se présentant sous forme de lotion

La composition de l'exemple 3 est la suivante :

| | |
|---|---|
| Eau | Qsp100% |
| Polyvinylpyrolidone (PVP K90) | 2% |
| Glycérol | 5% |

(suite)

| Eau | Qsp100% |
| Butylène glycol | 5% |
| Polysorbate 20 | 3% |
| Paraffinum liquidum | 4% |
| Dimethicone copolyol | 2% |
| Hydroxyethyl cellulose | 0,2% |
| Conservateur | 0,7% |
| Principe actif de l'exemple 1 | 0,5% |

## EVALUATION DE L'EFFICACITE SUR LES PARAMETRES DE LA DERMATITE ATOPIQUE

### TESTS IN VIVO

L'efficacité in vivo d'un principe actif obtenu à partir d'*Ophiopogon japonicus* a été testée lors d'une étude sur des enfants à peaux atopiques.

Les critères d'inclusion sont :

- Enfants de 4 mois à 4 ans présentant une dermatite atopique moyenne à modérée,
- Sujets ayant eu au moins une crise dans les 2 derniers mois,
- Sujets ayant reçu un traitement corticoïde pour soigner la dermatite atopique avant l'étude,
- Sujets n'utilisant pas d'antibiotique, anti-histaminique ou inhibiteur calcineurine depuis 3 semaines,
- Sujets n'ayant pas d'autres affections dermatologiques, ou maladie chronique ou évolutive.

Cette étude randomisée de 2 mois consiste en une étude simple aveugle d'une centaine de sujets, ayant été traités soit par une formule placebo, soit par une formule contenant le principe actif selon l'invention à 0.3%. (composition de l'exemple 2c).

Les sujets ont appliqué le produit 2 fois par jour sur la totalité du corps.

Les critères d'évaluation de l'efficacité sont l'index IDQOL (Infant's Dermatitis Quality of Life Index), le questionnaire DFI (Dermatitis Family Impact Questionnaire), le score SCORAD (Scoring Atopic Dermatitis). Ces critères ont été évalués par un médecin dermatologue à J0, J30 et J60.

Le protocole des visites est présenté dans le Tableau 4 suivant :

### Tableau 4

| | Visite 1 | Visite 2 | Visite 3 | Visite 4 |
|---|---|---|---|---|
| | Pré-inclusion J-10 | Inclusion J0 | Suivi J30 | Fin J60 |
| Nbre de sujets inclus | 104 sujets | 90 sujets | 85 sujets | 68 sujets |
| Groupe placebo | | 45 sujets | 43 sujets | 33 sujets |
| Groupe PA | | 45 sujets | 42 sujets | 35 sujets |
| Nbre de sujets exclus | 0 | 14 sujets | + 5 sujets | + 17 sujets |

L'exposition cutanée (SED) des enfants atopiques au principe actif selon l'invention est déterminée selon la formule suivante :

$$SED = [QA\ (g/j) \times 1000\ mg/g \times C(\%)/100 \times DAp\ (\%)\ /\ 100\ ]\ /\ poids\ corporel$$

SED : Systemic Exposure Dosage
QA : Quantité de produit appliquée par voie cutanée
C : Concentration de la substance étudiée dans le produit fini sur la zone d'application DAp : Absorption dermique exprimée en pourcentage de la dose d'essai supposée être appliquée dans les conditions réelles

Les enfants exposés ont entre 4 mois et 4 ans. Pour le calcul de l'exposition, la population cible est la population des nourrissons. En effet afin de maximiser la valeur de l'exposition, les nourrissons représentent la population la plus sensible du fait d'un rapport surface / poids corporel plus important que celui de l'adulte ou des enfants.

La quantité de produit appliquée par voie cutanée est de 1,1 g/j pour une surface corporelle de 2200cm$^2$ pour un produit corps et un poids de 3,4kg.

L'absorption cutanée du principe actif n'étant pas fixée, une valeur de 50% sera retenue, d'après les recommandations du SCCS 9th révision (septembre 2015).

On considère que le principe actif sera utilisé à une dose 0.3% dans le produit cosmétique fini, ce qui équivaut à une concentration de 0,03% dans le produit fini.

$$\text{SEDnourrisson} = [1{,}1 \times 1000 \times 0{,}03\%/100 \times 50\%/100] / 3{,}4 = 0{,}048 \text{ mg/kg pc/jour}$$

∘ <u>EFFET SUR LE TAUX DE RECHUTE</u>

Le taux de rechute a été analysé pour les deux groupes après 60 jours. Il est présenté dans le Tableau 5 ci-dessous.

**Tableau 5**

|  | Taux de rechute (%) | Taux sans rechute (%) |
|---|---|---|
| Placebo | 40 | 60 |
| Formulation de l'exemple 2c (Invention) | 21 | 79 |

On constate que dans les conditions de l'étude, après 60 jours de traitement biquotidien sur tout le corps, le taux de rechute avec le traitement selon l'invention est deux fois moins important qu'avec le traitement placebo.

∘ <u>EFFET SUR LE SCORE DE GRAVITE DE LA DERMATITE ATOPIQUE (SCORAD)</u>

Le SCORAD ou score de gravité de la dermatite atopique a été créé et validé en 1990 par un groupe d'experts : l'European Task Force of Atopic Dermatitis. C'est un outil de référence de suivi et d'évaluation de la pathologie par les médecins. Le SCORAD est défini par l'analyse de différents items : zone moyenne des lésions atopiques, érythème, œdème/papules, suintement/croûtes, excoriation, lichenification, sécheresse sur zone non lésée, prurit, insomnie.

L'analyse des SCORAD a été réalisée par le dermatologue chez les patients n'ayant pas observé de rechute pendant l'étude (60% des patients traités avec le placebo, 79% des patients traités avec le PA).

Les résultats sont présentés dans le Tableau 6 :

**Tableau 6**

|  | Variation du SCORAD (%) | |
|---|---|---|
|  | J30/J0 | J60/J0 |
| Placebo | -11 | -13 |
| Formulation de l'exemple 2c | -22 | -57 |

On constate que dans les conditions de l'étude, après 60 jours de traitement biquotidien du corps entier, la diminution du score de gravité de la dermatite atopique observée par le dermatologue est de -13% pour le placebo et -57% pour la formule contenant un principe actif obtenu à partir d'*Ophiopogon japonicus.*

On constate que dans les conditions de l'étude, après 60 jours de traitement, le score de gravité de la dermatite atopique du groupe traité par le principe actif obtenu à partir d'*Ophiopogon japonicus* est inférieur à celui du groupe traité par le placebo.

L'analyse des différents items du SCORAD a aussi été réalisée après 30 jours et après 60 jours est présentée dans le Tableau 7 :

**Tableau 7**

| | SCORAD à J30 | | SCORAD à J60 | |
|---|---|---|---|---|
| | Placebo | Principe actif | Placebo | Principe actif |
| Aire moyenne des lésions atopiques | 0.54 | 0.56 | 0.54 | 0.22 |
| Erythème | 0.15 | 0.13 | 0.23 | 0.03 |
| OEdème / papules | 0.12 | 0.03 | 0.00 | 0.03 |
| Suitement / croûtes | 0.00 | 0.00 | 0.00 | 0.00 |
| Excoriation | 0.00 | 0.00 | 0.00 | 0.00 |
| Lichenification | 0.19 | 0.31 | 0.19 | 0.13 |
| Sécheresse des zones non lésées | 0.50 | 0.38 | 0.35 | 0.28 |
| Prurit | 0.58 | 0.31 | 0.62 | 0.25 |
| Insomnie | 0.54 | 0.41 | 0.31 | 0.16 |

Une amélioration significative est observée en particulier sur les paramètres d'évaluation de l'érythème et du prurit.

∘ EVALUATION GENERALE DES TRAITEMENTS DES DERMATOLOGUES

Les deux traitements sont évalués par les dermatologues sur plusieurs critères : effet adoucissant, réduction de l'intensité des poussées, réduction de la fréquence des poussées, la bonne tolérance du produit et la satisfaction globale du produit.

Les résultats après 30 jours et 60 jours de traitement sont présentés dans le Tableau 8 suivant :

**Tableau 8**

| | Taux d'appréciation favorable (%) | | | |
|---|---|---|---|---|
| | J30 | | J60 | |
| **Appréciation des dermatologues** | Placebo | Principe actif | Placebo | Principe actif |
| Effet adoucissant | 98 | 95 | 85 | 97 |
| Réduction de l'intensité des poussées | 86 | 95 | 94 | 100 |
| Réduction de la fréquence des poussées | 86 | 88 | 94 | 100 |
| Bonne tolérance du produit | 100 | 95 | 91 | 97 |
| Satisfaction générale du produit | 86 | 90 | 91 | 97 |

On constate que dans les conditions de l'étude, après 30 jours de traitement, l'évaluation générale réalisée par les dermatologues sur le groupe traité avec le principe actif obtenu à partir d'*Ophiopogon japonicus* est généralement meilleure que celle du groupe traité par le placebo.

Cet effet continue après 60 jours de traitement ; le principe actif obtenu à partir d'un hydrolysat d'*Ophiopogon japonicus* montre un effet adoucissant, réduit l'intensité de poussées, la fréquence des poussées, significativement supérieur à ceux observés sur le groupe traité avec le placebo. Il est globalement satisfaisant.

∘ EFFET SUR LA QUALITE DE VIE

La dermatite atopique est une maladie chronique qui retentit sur la qualité de vie et qui nécessite des soins quotidiens. Un des suivis importants de l'évolution de la dermatite atopique se focalise sur les paramètres relatifs à la qualité de vie : importance de l'eczéma, dépréciation de l'index de qualité de vie de l'enfant (IDQoL), dépréciation de l'index de la qualité de vie de la famille (DFIQ), index total de la qualité de vie.

Ces paramètres ont été évalués sur les patients n'ayant pas eu de rechute pendant l'étude (placebo n=26 et PA n=32).

Les résultats après 30 jours et 60 jours de traitement sont présentés dans le Tableau 9 suivant.

**Tableau 9**

| Variation (%) | J30 / J0 | | J60 / J0 | |
|---|---|---|---|---|
| | Placebo | Principe actif | Placebo | Principe actif |
| Importance de l'eczéma | -27 | -50 | -27 | -89 |
| Index de qualité de vie de l'enfant (IDQoL) | 5 | -38 | -11 | -59 |
| Index de qualité de vie de la famille (DFIQ) | -11 | -36 | -25 | -51 |
| Index total de la qualité de vie (Index total QoL) | -5 | -38 | -19 | -58 |

Dans les conditions de l'étude, après 30 jours de traitement, une amélioration de la qualité de vie QoL est observée pour le groupe traité avec le principe actif obtenu à partir d'*Ophiopogon japonicus.*

Cet effet continue après 60 jours de traitement et l'amélioration est significativement visible sur les paramètres :

- Importance de l'eczéma : réduction de 89%
- Amélioration de l'index de qualité de vie de l'enfant (IDQoL) : réduction de 59%
- Amélioration de l'index de qualité de vie de la famille (DFIQ) : réduction de 51%
- Amélioration de l'index total de la qualité de vie (Index total QoL) : réduction de 58%.

∘ EVALUATION GENERALE DES TRAITEMENTS PAR LES PATIENTS

Les deux traitements sont évalués par les patients sur plusieurs critères : effet retardé de la rechute, confort journalier, produit bien toléré, utilisation agréable du produit, plaisir d'utiliser le produit, produit satisfaisant, efficacité du produit.

Les résultats après 30 jours et 60 jours de traitements sont présentés dans le Tableau 10 suivant :

**Tableau 10**

| Variation (%) | J30 / J0 | | J60 / J0 | |
|---|---|---|---|---|
| | Placebo | Principe actif | Placebo | Principe actif |
| Effet retardé des rechutes | 63 | 76 | 79 | 91 |
| Confort journalier | 72 | 71 | 76 | 80 |
| Produit bien toléré | 84 | 81 | 82 | 86 |
| Utilisation agréable du produit | 65 | 69 | 70 | 74 |
| Plaisir d'utiliser le produit | 67 | 76 | 73 | 77 |
| Satisfaction générale du produit | 72 | 86 | 79 | 89 |
| Le produit est-il efficace ? | 81 | 95 | 76 | 97 |

Dans les conditions de l'étude, après 30 jours de traitement, l'évaluation générale des patients sur le groupe traité avec le principe actif obtenu à partir d'*Ophiopogon japonicus* est généralement meilleure que celle du groupe traité par le placebo.

Cet effet continue après 60 jours de traitement ; le principe actif obtenu à partir d'*Ophiopogon japonicus* est significativement satisfaisant et efficace par comparaison au placebo.

En conclusion, les dermatologues considèrent que le principe actif obtenu à partir d'*Ophiopogon japonicus* selon l'invention a démontré un effet adoucissant, a réduit l'intensité et la fréquence des poussées. Il est donc un produit globalement satisfaisant. Il réduit le taux de rechute chez les enfants atopiques, et diminue significativement de 25% après 60 jours de traitement l'index SCORAD des enfants atopiques.

Les patients considèrent que le principe actif selon l'invention apporte une sensation de confort. Ils perçoivent une efficacité générale et considèrent le produit comme globalement satisfaisant.

Surtout, le principe actif selon l'invention réduit l'importance de l'eczéma et améliore la qualité de vie des enfants et de leurs familles.

## TESTS IN VITRO ET EX VIVO

Pour la mise en œuvre des tests in vitro et ex vivo, un modèle cellulaire, deux modèles d'épiderme humain reconstruit (REh) spécifiques et un modèle d'épiderme canin reconstruit (REc) spécifique ont été utilisés.

Le modèle cellulaire 2D correspond à une culture de kératinocytes humains normaux, soumis à une inflammation provoquée par un cocktail inflammatoire (Poly I :C + TNF$\alpha$ + IL-4).

Des kératinocytes humains normaux sont cultivés dans le milieu de culture complet pendant plusieurs jours. Le principe actf selon l'invention peut être ajouté dans le milieu de culture des cellules.

Les modèles d'épidermes reconstruits humains ont été décrits dans la publication du British Journal of Dermatology (2015) 173, 1006-1014.

Le modèle 3D d'épiderme humain reconstruit inflammé (REhI) mime l'inflammation de la dermatite atopique humaine.

Le modèle 3D d'épiderme humain reconstruit inflammé ayant une barrière altérée (REhBAI) mime l'inflammation de la dermatite atopique humaine sur un épiderme altéré.

Ces modèles humains 3D REhI et REhBAI ont été réalisés comme suit.

Des kératinocytes humains normaux sont cultivés en monocouches avec un milieu spécifique. Les modèles REhI et REhBAI sont obtenus par ensemencement de kératinocytes humains normaux sur des inserts de culture. Les cellules sont cultivées dans un milieu complet pendant plusieurs jours.

Pour le modèle REhI

Au 14$^{ème}$ jour de culture des REh, l'inflammation est provoquée par l'ajout d'un cocktail inflammatoire (Poly I/C + TNF$\alpha$ + IL-4 + IL-13) dans le milieu de culture pendant 2 jours supplémentaires.

Pour le modèle REhBAI

Au 15$^{ème}$ jour de culture des REh, les REh sont traités en topique par une solution de SDS (sodium dodécyl sulfate), afin d'obtenir une altération significative de la barrière cutanée. L'inflammation est provoquée par l'ajout d'un cocktail inflammatoire (Poly I/C + TNF$\alpha$ + IL-4 + IL-13) dans le milieu de culture pendant un jour supplémentaire.

Ces deux modèles humains sont comparés à un modèle d'épiderme reconstruit (REh) normal n'ayant été traité ni par le cocktail inflammatoire ni par le SDS. Le principe actif selon l'invention peut être ajouté par voie systémique dans le milieu de culture des REh, soit par voie topique sur l'épiderme reconstruit.

Le modèle 3D d'épiderme canin reconstruit inflammé (REcI) mime l'inflammation de la dermatite atopique canine, il a été réalisé comme suit :

Après ensemencement de kératinocytes canins normaux sur des inserts de culture, les cellules sont cultivées dans un milieu complet pendant plusieurs jours.

Au 11$^{ème}$ jour de culture des REc, l'inflammation est provoquée par l'ajout d'un cocktail inflammatoire (Poly I/C + TNF$\alpha$ + IL-4 + IL-13) dans le milieu de culture pendant 2 jours supplémentaires.

Ce modèle est comparé à un modèle d'épiderme canin reconstruit (REc) normal qui n'a pas été traité avec le cocktail inflammatoire. Le principe actif selon l'invention peut être ajouté par voie systémique dans le milieu de culture des REc.

Les effets du principe actif selon l'invention, ont été évalués :

- sur l'inflammation de la peau atopique,
- sur la fonction barrière cutanée,
- sur le microbiote cutané

### 1/ Effet sur l'inflammation de la peau atopique

La peau est constamment soumise à des signaux provenant de l'environnement. Ces signaux vont activer des mécanismes de défense notamment immunitaires qui se traduisent par une réaction inflammatoire (Skabytska et al., « The role of innate immune signaling in the pathogenesis of atopic dermatitis and consequences for treatments, Semin Immunopathol », 38, 29-43, 2016).

Le système immunitaire cutané présente deux réponses : innée et adaptative. La réaction la plus efficace va être conférée par un équilibre entre ces deux réponses alors qu'un déséquilibre est à l'origine de la dermatite atopique. De façon schématique, il existe deux sous-populations lymphocytaires, les lymphocytes Th1 et Th2. Les lymphocytes Th1 favorise une réponse dite cellulaire tandis que les Th2 orientent vers une réponse humorale. Lorsque la réponse Th2 prend le pas sur la Th1, le développement d'allergies est alors favorisé car la production d'immunoglobulines IgE est massivement augmentée.

En raison de la porosité de la barrière épidermique qui apparaît au cours de cette pathologie, divers éléments exogènes tels que les allergènes vont pouvoir pénétrer dans l'épiderme. Ces allergènes vont entraîner une réponse immunitaire qui se traduit par la sécrétion de cytokines par les kératinocytes telles que les interleukines 33 et 25 mais aussi la lymphopoïétine stromale thymique (TSLP). Ces cytokines enclenchent alors une cascade d'activation de différentes cellules qui aboutit à l'orientation vers une réponse Th2. Cette réponse est caractérisée par la sécrétion des interleukines 4, 5, 13 et 31 mais aussi par la production d'IgE (Agrawal et al., « Skin barrier defects in atopic dermatitis », Current allergy Asthma Rep, 14, 1-11, 2014). En retour, ces cytokines vont avoir des effets néfastes sur la fonction barrière en altérant le bon déroulement de la différenciation épidermique.

∘ EFFET SUR LES MARQUEURS DE L'INFLAMMATION

Cette étude consiste à évaluer l'effet d'un principe actif obtenu à partir *d'Ophiopogon japonicus* sur les marqueurs de l'inflammation (IL-8 et/ou TSLP) sur le modèle cellulaire 2D, les modèles humains REhl et REhBAI et sur le modèle canin REcl.

Le principe actif a été ajouté en traitement systémique, soit à 0.10%, 0.15% ou 0.25% directement dans les milieux de culture.

Amanaka *et al* (2011) et Stalder *et al* (2014) et Klukowska-Rötzler et al (2013) ont expliqué dans leurs publications respectives (« The role of cytokines/chemokines in the pathogenesis of atopic dermatitis », Curr Probl Dermatol. 2011;41:80-92*, «* Fragility of epidermis and its consequence in dermatology », J Eur Acad Dermatol Venereol. 2014 Jun;28 Suppl 4:1-18, *«* Expression of thymic stromal lymphopoietin in canine atopic dermatitis », Vet Dermatol. 2013 Feb;24(1):54-9.e13-4) que les peaux atopiques humaines et canines sont caractérisées principalement par une surexpression des molécules pro-inflammatoires comme les IL-4, IL-8, IL-13 et le lymphopoeitine stromale thymique (TSLP).

La réponse inflammatoire dans le modèle cellulaire 2D est évaluée par mesure de la teneur en lymphopoïétine stromale thymique (TSLP) libérées dans le milieu de culture.

La réponse inflammatoire dans les modèles 3D humains REhl et REhBAI et le modèle 3D canin REcl est évaluée par mesure de la teneur en interleukine IL-8 et/ou en lymphopoïétine stromale thymique (TSLP) libérées dans les sous-nageants tissulaires.

Le milieu de culture 2D et les sous-nageants des RE sont collectés et conservés à -20°C. Les sécrétions en IL-8 et/ou TSLP sont mesurées au moyen du kit Elisa.

Les résultats sont comparés à une culture cellulaire ou aux modèles normaux REh ou REc n'ayant pas été en contact avec le cocktail inflammatoire.

Les résultats sur le modèle 2D sont présentés dans le Tableau 11, ceux sur les modèles humains dans le Tableau 12 et ceux sur le modèle canin dans le Tableau 13.

***Tableau 11***

|  | TSLP (fg/$\mu$g protéines) | Efficacité / Témoin inflammé (%) |
| --- | --- | --- |
| 2D normal |  |  |
| Témoin | 1 |  |
| 2D inflammé |  |  |
| Témoin | 633 |  |
| Exemple 1 à 0.15% | 434 | -31% |

On constate que le cocktail inflammatoire provoque bien un effet inflammatoire (libération de TSLP) dans le modèle cellulaire.

Le principe actif obtenu à partir d'un hydrolysat d'*Ophiopogon japonicus* permet de réduire l'inflammation provoquée, en diminuant de 31% la libération de TSLP dans des cultures de kératinocytes humains.

*Tableau 12*

|  | TSLP (pg/ml) | Efficacité / Témoin inflammé (%) | IL-8 (pg/ml) | Efficacité / Témoin inflammé (%) |
|---|---|---|---|---|
| REh normal |  |  |  |  |
| Témoin | 0 |  | 24 |  |
| Exemple 1 à 0.25% | 0 |  | 24 |  |
| REhl |  |  |  |  |
| Témoin | 253 |  | 622 |  |
| Exemple 1 à 0.10% | 204 | -19% | 569 | -9% |
| Exemple 1 à 0.25% | 189 | -25% | 489 | -21% |
| REhBAI |  |  |  |  |
| Témoin | 30 |  | 794 |  |
| Exemple 1 à 0.10% | 15 | -50% | 621 | -22% |
| Exemple 1 à 0.25% | 14 | -54% | 603 | -24% |

On constate que le cocktail inflammatoire provoque bien un effet inflammatoire (libération de TSLP et IL-8) dans les modèles REhl et REhBAI.

Le principe actif obtenu à partir d'*Ophiopogon japonicus* n'a pas d'effet négatif sur le modèle REh normal, il ne provoque pas d'inflammation.

Dans le modèle REhl, le principe actif obtenu à partir d'*Ophiopogon japonicus* permet de réduire l'inflammation provoquée, en diminuant de 25% la teneur en TSLP et de 21% la teneur en IL-8.

De même, dans le modèle REhBAI, le principe actif obtenu à partir d'*Ophiopogon japonicus* permet de réduire l'inflammation provoquée, en diminuant de 24% la teneur en IL-8. L'utilisation d'un principe actif obtenu à partir d'*Ophiopogon japonicus* permet donc de limiter l'inflammation sur les zones lésées et sur les zones non lésées des peaux atopiques.

*Tableau 13*

|  | TSLP (pg/ml) | Efficacité / Témoin inflammé (%) |
|---|---|---|
| REc normal |  |  |
| Témoin | 15 |  |
| REcl |  |  |
| Témoin | 46 |  |
| Exemple 1 à 0.25% | 19 | -59% |

On constate que le cocktail inflammatoire provoque bien un effet inflammatoire (libération de TSLP) dans le modèle REcl.

Dans le modèle REcl, le principe actif obtenu à partir d'un hydrolysat d'*Ophiopogon japonicus* permet de réduire l'inflammation provoquée, en diminuant de 59% la teneur en TSLP.

○ EFFET SUR LES GENES ASSOCIES A L'INFLAMMATION

L'étude consiste à évaluer l'effet d'un principe actif obtenu à partir d'*Ophiopogon japonicus* sur les gènes associés à l'inflammation.

Le principe actif a été ajouté en traitement systémique, soit à 0.10 et 0.25% directement dans le milieu de culture des REh.

En 2011, Kamsteeg a identifié dans sa publication (« Type 2 Helper T-Cell Cytokines induce morphologic and Molecular characteristics of Atopic Dermatitis in Human Skin Equivalent », Journal of investigative dematology, 127, 1786-1789, 2007), que les gènes clés associés à l'inflammation sont anhydrase carbonique II (CAII) et équivalent de facteur de croissance épidermique neutre (NELL2) dans les lésions des peaux atopiques en comparaison avec du psoriasis.

Le protocole de la présente étude est décrit en suivant.

Les ARN totaux des RE sont extraits par un kit RNeasy. La quantification et l'évaluation de la qualité des ARN isolés sont réalisées en utilisant un spectrophotomètre NanoDrop et un bioanalyseur Agilent.

La fluorescence des signaux est détectée par un scanner spécifique et l'analyse des images est réalisée par un logiciel.

L'analyse des process biologiques est réalisée sur les données transcriptomiques de la base de données DAVID.

1730 gènes des 20 000 gènes du modèle REhl ont été surexprimés par rapport au modèle REh normal, et 2086 gènes ont été sous-exprimés.

Quatre voies regroupent ces 3816 gènes : l'adhésion cellulaire, la migration cellulaire, l'inflammation et la différenciation cellulaire épidermique. Les gènes (CXCL8, CA2, NELL2, TLR2, TLR3, NOTCH, STAT2, SPINK5, PLAUR et IL1A) associés à l'inflammation sont surexprimés dans le REI.

Les résultats obtenus sont présentés dans le Tableau 14 ci-après.

*Tableau 14*

| | NELL2 (%) | Efficacité / Témoin normal (%) | Tenascine C (TNC) (%) | Efficacité / Témoin normal (%) |
|---|---|---|---|---|
| REh normal | | | | |
| Témoin | 100 | | 100 | |
| Exemple 1 à 0.25% | 98 | | 122 | |
| REhl | | | | |
| Témoin | 147 | | 396 | |
| Exemple 1 à 0.25% | 120 | -57% | 380 | -5% |
| Exemple 1 à 0.5% | 114 | -70% | 284 | -38% |

On constate que le cocktail inflammatoire provoque bien un effet inflammatoire important (surexpressions des gènes NELL2 Tenascine C) dans le modèle REhl.

Le principe actif obtenu à partir d'*Ophiopogon japonicus,* dans le modèle REhl, permet de réduire l'expression de NELL2 et celle de Tenascine C. En particulier le principe actif testé à 0.5% permet de réduire de 70% l'expression de NELL2 et de 38% celle de Tenascine C. L'utilisation d'un principe actif obtenu à partir d'un hydrolysat d'*Ophiopogon japonicus* permet donc de limiter l'expression des gènes de l'inflammation caractéristique de la dermatite atopique.

### 2/ *Effet sur la fonction barrière cutanée*

La peau forme une barrière contre les agressions environnementales et la perte d'eau. Au cours de la dermatite atopique, une rupture de la fonction barrière a été mise en évidence. Divers paramètres sont altérés, des protéines structurales aux lipides en passant par les jonctions intercellulaires.

La filaggrine est une protéine indispensable à la formation du stratum cornéum. Elle est responsable de la réticulation des protéines de différenciation telles que les kératines et consécutivement du passage des kératinocytes en cornéocytes. Cette protéine confère au stratum cornéum sa robustesse, et par conséquent ses propriétés barrières vis-à-vis de la perte d'eau ou des agressions extérieures.

Dans le cas de la dermatite atopique, on sait que le gène codant pour la filaggrine peut présenter des mutations induisant une réduction de sa synthèse. Au-delà de cette mutation, l'inflammation associée à cette pathologie a également des conséquences négatives sur la synthèse de filaggrine, mais aussi sur celle de la loricrine, de l'involucrine et de la claudine-1. L'intégrité de la barrière épidermique est alors altérée permettant la pénétration d'agents indésirables.

○ EFFET SUR UN MARQUEUR DE LA COHESION EPIDERMIQUE

L'étude consiste à évaluer l'effet d'un principe actif obtenu à partir d'*Ophiopogon japonicus* sur la cohésion épidermique sur le modèle REhBAI.

Après 17 jours de culture des REh à fonction barrière altérée, les épidermes sont fixés, déshydratés avec 4% de paraformaldéhyde et inclus en paraffine. Des coupes de 4 $\mu$m sont réalisées à l'aide d'un microtome. L'analyse morphologique des coupes est faite par coloration HE (hematoxyline éosine).

L'analyse immunohistofluorescence de la claudine-1 dans le modèle 3D humain est réalisée après incubation d'un anticorps primaire et d'un anticorps secondaire. La visualisation est réalisée sur un microscope couplé à un système d'analyse d'images. L'analyse quantitative est réalisée avec un logiciel MatLab®.

Les résultats sont comparés à un modèle REh normal, n'ayant pas été en contact avec le cocktail inflammatoire.

Le principe actif de l'exemple 1 a été ajouté en traitement topique, soit à 0.075 et 0.150% directement dans le milieu de culture des REh.

Les résultats obtenus sont présentés dans le Tableau 15 ci-dessous.

**Tableau 15**

|  | Claudine-1 (%) | Efficacité Claudine-1/ Témoin normal (%) |
|---|---|---|
| REhBAI normal |  |  |
| Témoin | 100 |  |
| REhBAI |  |  |
| Témoin | 38 |  |
| Exemple 1 à 0.075% | 43 | 29% |
| Exemple 1 à 0.15% | 48 | 59% |

On constate que le cocktail inflammatoire provoque bien une limitation de la cohésion en limitant la synthèse de claudine-1 dans le modèle REhBAI.

Dans le modèle REhBAI, le principe actif obtenu à partir d'*Ophiopogon japonicus* permet de maintenir la synthèse du marqueur de la cohésion épidermique. En particulier testé à 0.15%, il permet par voie topique, d'augmenter la synthèse de claudine-1 de 59%

L'utilisation d'un principe actif obtenu à partir d'un hydrolysat d'*Ophiopogon japonicus* en traitement topique permet donc d'augmenter la synthèse des marqueurs de cohésion épidermique sur les peaux atopiques.

○ EFFET SUR LES MARQUEURS DE DIFFERENCIATION EPIDERMIQUE

L'étude consiste à évaluer l'effet d'un principe actif obtenu à partir d'*Ophiopogon japonicus* sur les marqueurs de différenciation sur le modèle REhl et/ou sur le modèle REhBAI.

Tous les symptômes de la dermatite atopique sont associés à la perturbation de la fonction de barrière cutanée des patients atopiques. Plusieurs publications, comme Agrawal et al 2014 et Feingold et al 2014 (« Role of lipids in the formation and maintenance of the cutaneous permeability barrier » Biochim Biophys Act mars 2014, 1841(3) 280-94), expliquent que les synthèses des protéines de différenciation (loricrine, involucrine et/ou filaggrine) sont sous-exprimées dans les peaux atopiques.

L'impact de la réponse inflammatoire de l'épiderme reconstruit est évalué sur des marqueurs de différenciation, la loricrine et/ou la filaggrine.

Après 13 jours de culture des REcl ou 17 jours de culture des REhBAI, les épidermes sont fixés, déshydratés avec 4% de paraformaldéhyde et inclus en paraffine. Des coupes de 4 μm sont réalisées à l'aide d'un microtome. L'analyse morphologique des coupes est faite par coloration HE (hematoxyline éosine).

L'analyse immunohistofluorescence de la filaggrine et/ou la loricrine dans les modèles 3D humains et canins est réalisée après incubation d'un anticorps primaire spécifique et d'un anticorps secondaire. La visualisation est réalisée sur un microscope couplé à un système d'analyse d'images. L'analyse quantitative est réalisée avec un logiciel MatLab®.

Les résultats sont comparés à un modèle RE normal, n'ayant pas été en contact avec le cocktail inflammatoire.

Le principe actif de l'exemple 1 a été ajouté :

- soit en traitement systémique, à 0.10% et 0.25%, directement dans le milieu de culture des REh et REc,
- soit en traitement topique, à 0.15% , directement sur les REh

Les résultats obtenus sur les modèles humains sont présentés dans les Tableau 16 et 17 pour les traitements systémiques sur les REh ou REc (traitement systémique) et le Tableau 18 pour le traitement topique des REh, ci-dessous.

Traitement systémique

17

### Tableau 16

|  | Intensité de fluorescence de la filaggrine / surface (UA) | Capacité à maintenir la synthèse de filaggrine (%) |
|---|---|---|
| REh normal |  |  |
| Témoin | 33 |  |
| Exemple 1 à 0.25% | 32 |  |
| REhl |  |  |
| Témoin | 13 |  |
| Exemple 1 à 0.10% | 18 | 25% |
| Exemple 1 à 0.25% | 22 | 45% |
| REhBAl |  |  |
| Témoin | 11 |  |
| Exemple 1 à 0.10% | 16 | 23% |
| Exemple 1 à 0.25% | 18 | 32% |

### Tableau 17

|  | Intensité de fluorescence de la loricrine / surface (UA) | Capacité à maintenir la synthèse de loricrine (%) |
|---|---|---|
| REc normal |  |  |
| Témoin | 19 |  |
| REcl |  |  |
| Témoin | 1 |  |
| Exemple 1 à 0.25% | 9 | 44% |

Traitement topique

### Tableau 18

|  | Intensité de fluorescence de la filaggrine / surface (UA) | Capacité à maintenir la synthèse de filaggrine (%) | Intensité de fluorescence de la loricrine / surface (UA) | Capacité à maintenir la synthèse de loricrine (%) |
|---|---|---|---|---|
| REh normal |  |  |  |  |
| Témoin | 23 |  | 29 |  |
| REhBAl |  |  |  |  |
| Témoin | 17 |  | 0 |  |
| Exemple 1 à 0.15% | 32 | +250% | 6 | +21% |

On constate que le cocktail inflammatoire provoque bien une limitation de la différenciation (inhibition de la synthèse de filaggrine et/ou de la loricrine) dans les modèles humains et canin, quel que soit le traitement systémique ou topique.

Le principe actif n'a pas d'effet sur le modèle REh normal, il n'augmente pas les marqueurs de différenciation.

Dans les modèles humain et canin REhl et REcl, le principe actif obtenu à partir d'*Ophiopogon japonicus* permet, par voie systémique, de maintenir la synthèse des marqueurs de différenciation. En particulier testé à 0.25%, il permet par voie systémique, d'augmenter la synthèse de filaggrine de 45% sur le modèle humain et de 44% sur le modèle canin.

Dans le modèle humain REhBAI, le principe actif selon l'invention permet, par voie systémique ou par voie topique, de maintenir la synthèse des marqueurs de la différenciation. En particulier, testé à 0.15%, il permet par voie topique, d'augmenter la synthèse de loricrine et de filaggrine.

L'utilisation d'un principe actif obtenu à partir d'un hydrolysat d'*Ophiopogon japonicus* en traitement systémique ou topique permet donc d'augmenter la synthèse des marqueurs de différenciation sur les peaux atopiques, qu'elles soient humaines ou animales.

∘ EFFET SUR LES GENES ASSOCIES A LA DIFFERENCIATION EPIDERMIQUE

L'étude consiste à évaluer l'effet d'un principe actif obtenu à partir d'*Ophiopogon japonicus* selon l'invention sur les gènes associés à la différenciation.

Le principe actif a été ajouté en traitement systémique, soit à 0.10 et 0.25% directement dans le milieu de culture des REh.

En 2005, Sugiura avait indiqué après analyse des 23 000 gènes de biopsies cutanées de patients atteints de dermatite atopique par comparaison à des contrôles, que 10 gènes présentent des différences importantes entre les patients et les contrôles. Les modifications importantes sont la sous-régulation des gènes codant pour la loricrine et la filaggrine. (Sugiura et al. "Large-scale DNA microarray analytsis of atopic lesions shows of on epidermis differentiation gene cluster in the alternative pathway and lack of protective gene expression in the cornified envelope" BJD, vol 152, Issue1, jan 2015, p146-149).

En 2014, Zhang a complété dans sa publication (« Screening for key genes associated with atopic dermatitis with DNA microarrays » Molecular Medecine Reports, mars 2014, vol 9 Issue 3, p1049-1055), que les gènes clés associés à la dermatite atopique sont liés à la différenciation : loricrine (LOR), kératine 17 (KRT17), petites protéines de répétition riche en proline (SPRRs) and involucrine (IVL).

Le protocole de l'étude est décrit en suivant.

Les ARN totaux des REh sont extraits par un kit RNeasy. La quantification et l'évaluation de la qualité des ARN isolés sont réalisées en utilisant un spectrophotomètre NanoDrop et un bioanalyseur Agilent.

La fluorescence des signaux est détectée par un scanner spécifique SureScan Microarrays et l'analyse des images est réalisée par un logiciel.

L'analyse des procédés biologiques est réalisée sur les données transcriptomiques de la base de données DAVID.

1730 gènes des 20 000 gènes du modèle REh inflammé ont été sur-exprimés par rapport au modèle RE normal, et 2086 gènes ont été sous-exprimés.

Quatre voies regroupent ces 3816 gènes : l'adhésion cellulaire, la migration cellulaire, l'inflammation et la différenciation cellulaire épidermique :

- Les gènes (TGM1, S100A8, S100A9 et KLK7) associés à la différenciation sont sur-exprimés dans le REh inflammé.

- Les gènes (FLG, LOR, KRT1, KRT10 et ITGA2) associés aussi à la différenciation sont sous-régulés dans le REh inflammé.

Les résultats sont présentés dans le Tableau 19 ci-dessous :

*Tableau 19*

| | Filaggrine (%) | Efficacité / Témoin normal (%) | Loricrine (%) | Efficacité / Témoin normal (%) | TGM1 (%) | Efficacité / Témoin normal (%) |
|---|---|---|---|---|---|---|
| REh normal | | | | | | |
| Témoin | 100 | | 100 | | 100 | |
| Exemple 1 à 0.25% | 112 | | 103 | | 96 | |
| REhl | | | | | | |
| Témoin | 61 | | 52 | | 437 | |
| Exemple 1 à 0.25% | 74 | 33% | 63 | 23% | 385 | -15% |
| Exemple 1 à 0.5% | 88 | 69% | 71 | 40% | 318 | -35% |

On constate que le cocktail inflammatoire provoque bien une limitation de la différenciation (sous expression du gène FLG, filaggrine et du gène LOR, loricrine et surexpression du gène TGM1, transglutaminase 1) dans le modèle REhl.

L'utilisation d'un principe actif obtenu à partir d'un hydrolysat d'*Ophiopogon japonicus* permet donc d'augmenter l'expression des gènes filaggrine et loricrine, et de diminuer l'expression du gène transglutaminase 1, associés à la différenciation.

∘ EFFET SUR L'ORGANISATION ET LA CONFORMATION DES LIPIDES EPIDERMIQUES

Au cours de la dermatite atopique, plusieurs mécanismes impliqués dans le processus de maturation des lipides épidermiques sont altérés. Les corps lamellaires, organites en charge du stockage des précurseurs lipidiques, présentent un défaut de maturation et de libération de leur contenu. La concentration en céramides est réduite en raison d'une activité anormale des enzymes du métabolisme lipidique. De plus, au-delà de l'aspect quantitatif, une réduction de la longueur des chaînes des acides gras et des céramides est observée entraînant une augmentation de la perméabilité de la barrière épidermique. Enfin, l'organisation des lipides entre eux est également altérée (Elias et al. «Lipid abnormalities and lipid-based repair strategies in atopic dermatitis" Biochimics et Biophyiscs acta, 1841, 323-330, 2014 ; Van Smeden et al. "The important role of stratum corneum lipids for the cutaneous barrier function", Biochemica et biophysica acta, 1841, 295-313, 2014).

Afin d'évaluer directement *in vivo* l'influence du principe actif selon l'invention sur la composante lipidique, une étude visant à établir la signature moléculaire des peaux atopiques a été réalisée par microspectroscopie Raman. Cette étude, menée à la fois sur des volontaires atteints de dermatite atopique et sur des volontaires sains, en mettant en évidence une perturbation de la conformation intramoléculaire des lipides ainsi qu'une modification de leur organisation chez les sujets atopiques.

Dans un deuxième temps, l'effet du principe actif selon l'invention sur l'organisation et la conformation des lipides a été mesuré *in vivo* après application biquotidienne pendant 60 jours. Cette étude a été réalisée sur 40 volontaires adultes atteints de dermatite atopique légère à modérée, répartis en deux groupes : l'un traité avec une formule placebo, l'autre avec une formule contenant le principe actif selon l'invention à 0,5%.

Les bandes spectrales caractéristiques de l'organisation et de la conformation des lipides ont été étudiées *via* les descripteurs suivants :

- le rapport vCC *trans* / vCC *gauche* donne des informations sur la conformation intramoléculaire des lipides. La prédominance de la conformation *trans* est reliée à une compacité supérieure de la barrière cutanée. Par contre, une quantité supérieure de conformères *gauche* traduit un affaiblissement de la compacité des édifices lipidiques cutanés.

- le rapport $\nu_{asym}CH_2/\nu_{sym}CH_2$ est un indicateur de l'organisation latérale des lipides du *stratum corneum.* Des valeurs élevées de ce rapport sont associées à une organisation ordonnée. Une réduction de ce rapport est associée à une perte d'organisation.

Le système expérimental utilisé pour mesurer ces paramètres est une microspectroscopie Raman. Il s'agit d'une sonde confocale Raman couplée à un spectromètre Raman dispersif, le spectromètre est équipé d'une caméra CCD (Coupled Charge Detector). Le sytème d'acquisition est contrôlé par un logiciel.

Les profils Raman sont enregistrés en collectant des spectres à partir de -10$\mu$m au-dessus de la surface de la peau et jusqu'à une profondeur de 40$\mu$m.

Cette étude a été réalisée en partenariat avec les équipes des professeurs Arlette Baillet-Guffroy (Groupe de Chimie Analytique Paris-Sud, EA4041, Université Paris-Sud 11) et Michel Manfait (MéDIAN-CNRS UMR 7369, Faculté de Pharmacie de Reims Champagne-Ardenne).

***Tableau 20***

| Variation / J0 | Conformation des lipides (Rapport vCC trans / vCC gauche) | Organisation des lipides (Rapport vasymCH2/vsym CH2) |
|---|---|---|
| Groupe Placebo | -42.5% | -15.3% |
| Groupe Formule contenant le principe actif selon l'invention | +71.8% | -4.1% |

Dans les conditions de cette étude, après 2 mois de traitement, la formule contenant le principe actif selon l'invention à 0,5% permet de préserver la qualité de la barrière cutanée.

Sur les sujets ayant utilisé la formule active :

- la prédominance de la conformation *trans* est observée (augmentation du rapport *trans* / *gauche*) et est reliée à une compacité supérieure de la barrière cutanée.
- l'organisation de la matrice lipidique du *stratum corneum* est maintenue dans son état optimal dans le temps (rapport $\nu_{asym}CH_2/ \nu_{sym}CH_2$ quasi-inchangé).

Sur les sujets ayant appliqué la formule placebo :

- une quantité supérieure de conformères *gauche* est constatée (diminution du rapport *trans* /*gauche*) avec le temps, traduisant un affaiblissement de la compacité des édifices lipidiques cutanés.
- une chute significative de l'organisation de la matrice lipidique est notée avec le temps (diminution du rapport $\nu_{asym}CH_2/ \nu_{sym}CH_2$).

∘ <u>EVALUATION DE L'INTEGRITE ET DE LA RESISTANCE DE LA BARRIERE EPIDERMIQUE</u>

L'intégrité et la résistance de la barrière épidermique peut être montrée au moyen de plusieurs études :

- une visualisation de l'état général de la construction épidermique d'un épiderme reconstruit,
- une quantification de l'intégrité de la barrière épidermique par l'évaluation de la non-pénétration d'un colorant ou la quantification des vésicules présentes dans l'épiderme,
- la mesure de la résistance transépithéliale caractérisant la résistance cutanée.

■ <u>VISUALISATION GENERALE DE LA CONSTRUCTION EPIDERMIQUE</u>

L'étude consiste à évaluer l'effet d'un principe actif obtenu à partir d'*Ophiopogon japonicus* sur la construction épidermique sur les modèles humains REhl et REhBAI et sur le modèle canin REcl.

La construction épidermique dans les modèles 3D inflammés est évaluée par l'analyse morphologique des coupes d'épidermes après coloration HE (hematoxyline-éosine).

Après 17 jours de culture des REh ou 13 jours des REc, les épidermes sont fixés, déshydratés avec 4% de paraformaldéhyde et inclus en paraffine. Des coupes de 4μm sont réalisées à l'aide d'un microtome. L'analyse morphologique des coupes est faite par coloration HE. La visualisation est réalisée sur microscope couplé à un système d'analyse d'images.

Le principe actif a été ajouté en traitement topique, à 0.05 et 0.1% dans une formule cosmétique sur les épidermes humains et à 0.25% sur les épidermes canins. La formule placebo a aussi été testée.

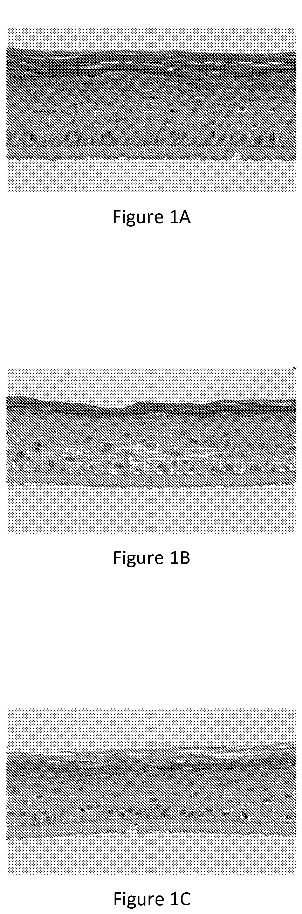

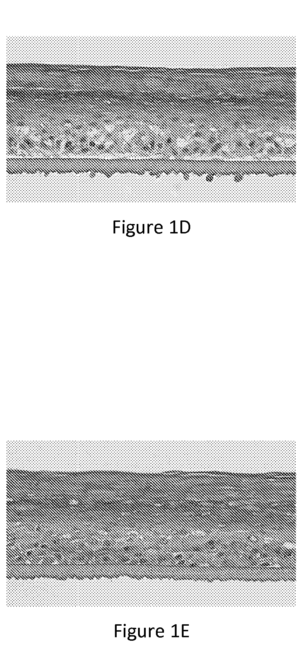

Les résultats sont présentés dans le Tableau 21 ci-dessous et sur les Figures 1A à 1E et ceux sur le modèle canin dans le Tableau 22.

*Tableau 21*

| | Morphologie générale |
|---|---|
| REh normal | |
| Témoin | +++ |
| Placebo | +++ |
| Exemple 2b contenant 0.1% du PA | +++ |
| REhl | |
| Témoin | - |
| Placebo | - |
| Exemple 2a contenant 0.05% du PA | + |
| Exemple 2b contenant 0.1% du PA | ++ |
| REhBAI | |
| Témoin | - |
| Placebo | - |

(suite)

| REhBAI | |
|---|---|
| Exemple 2a contenant 0.05% du PA | + |
| Exemple 2b contenant 0.1% du PA | ++ |

| +++ signifie épiderme reconstruit normal, couches épidermiques compactes<br>++ signifie épiderme reconstruit altéré, couches épidermiques moins compactes<br>+ signifie épiderme reconstruit altéré, couches épidermiques beaucoup moins compactes<br>- signifie épiderme reconstruit très altéré, couches épidermiques non compactes ou aspect spongieux de l'épiderme |
|---|

**Tableau 22**

| | Morphologie générale |
|---|---|
| REc normal | |
| Témoin | +++ |
| REcI | |
| Témoin | - |
| Exemple 2a contenant 0.25% du PA | ++ |

| +++ signifie épiderme reconstruit normal, couches épidermiques compactes<br>++ signifie épiderme reconstruit altéré, couches épidermiques moins compactes<br>+ signifie épiderme reconstruit altéré, couches épidermiques beaucoup moins compactes<br>- signifie épiderme reconstruit très altéré, couches épidermiques non compactes ou aspect spongieux de l'épiderme |
|---|

On constate que le cocktail inflammatoire provoque bien une perturbation importante de la morphologie globale de l'épiderme reconstruit, qu'il soit humain ou canin. Il apparait altéré et spongieux dans le témoin des modèles RE inflammés.

Par contre, le principe actif obtenu à partir d'*Ophiopogon japonicus* utilisé par voie topique permet de protéger la morphologie générale de l'ER dans les modèles REh et REc inflammés. L'utilisation d'un principe actif obtenu à partir d'un hydrolysat d'*Ophiopogon japonicus* permet par conséquent en application topique d'améliorer la morphologie générale des épidermes sur les peaux atopiques humaines ou canines.

▪ <u>EVALUATION DE L'INTEGRITE DE LA BARRIERE EPIDERMIQUE</u>

L'étude consiste à évaluer l'effet d'un principe actif obtenu à partir d'*Ophiopogon japonicus* selon l'invention sur l'intégrité de la barrière épidermique sur le modèle humain REhBAI. Deux tests permettent de visualiser l'intégrité de la fonction barrière :

- L'utilisation d'une sonde fluorescente, le colorant lucifer yellow. Plus la coloration est intense, plus la barrière épidermique est importante.
- L'utilisation de la spongiose épidermique, qui permet la quantification des vésicules dans l'épiderme. Plus la spondiose épidermique est quantifiée, plus la présence de vésicules est observée, plus la barrière épidermique est altérée.

Après plusieurs jours de culture des REh, le lucifer yellow sont déposés sur les épidermes reconstruits. Après une incubation à 37°C, les épidermes sont rincés avec du tampon PBS et fixés avec du PFA. Des coupes $4\,\mu$m sont ensuite réalisées à l'aide d'un microtome.

La visualisation de l'intégrité de la barrière cutanée est réalisée sur microscope couplé à un système d'analyse d'image.

L'épaisseur des épidermes et la pénétration du lucifer yellow ont été mesurées sur les coupes histologiques réalisées.

Le principe actif a été ajouté en traitement topique, à 0.5 et 1% dans une formule cosmétique sur les épidermes. La formule placebo a aussi été testée.

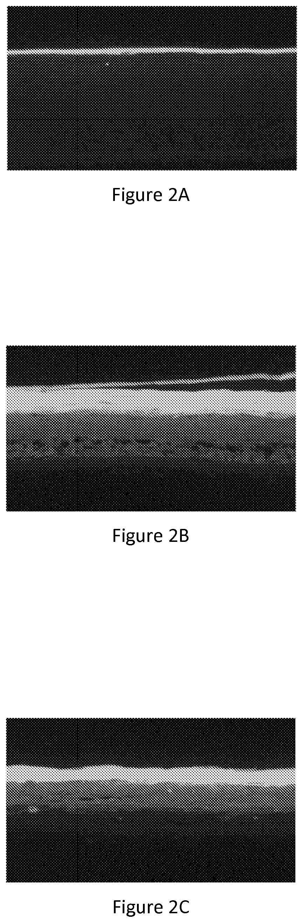

Les résultats sont présentés dans le Tableau 23 ci-dessous et sur les Figures 2A à 2C.

*Tableau 23*

| | Mesure de pénétration du lucifer yellow ($\mu$m) | Efficacité / Témoin REhBAI | Mesure de spondiose épidermique (%) | Efficacité / Témoin REhBAI |
|---|---|---|---|---|
| REh normal | | | | |
| Témoin | 4 | | 0 | |
| Placebo | 4 | | | |
| Exemple 2b contenant 0.1% du PA | 4 | | | |
| REhBAI | | | | |
| Témoin | 85 | | 9.6 | |
| Placebo | 65 | -25% | | |
| Exemple 2a contenant 0.05% du PA | 56 | -36% | | |
| Exemple 2b contenant 0.075% du PA | | | 1.2 | -88% |
| Exemple 2b contenant 0.10% du PA | 51 | -42% | | |
| Exemple 2b contenant 0.15% du PA | | | 0.4 | -96% |

La dégradation de l'intégrité de la barrière épidermique est très importante sur le modèle REhBAI, la pénétration du colorant lucifer yellow et la mesure de spongiose épidermique sont augmentés.

On constate que le principe actif obtenu à partir d'un hydrolysat d'*Ophiopogon japonicus* limite la pénétration du lucifer yellow et la présence de la spongiose épidermique, donc il protège l'intégrité de la barrière épidermique dans le modèle REhBAI.

L'utilisation d'un principe actif obtenu à partir d'un hydrolysat d'*Ophiopogon japonicus* permet par conséquent, en application topique ou systémique, de protéger l'intégrité de la barrière épidermique des peaux atopiques.

▪ EVALUATION DE LA RESISTANCE DE LA BARRIERE CUTANEE

L'étude consiste à évaluer la résistance de la barrière cutanée d'un épiderme reconstruit en présence ou non d'un principe actif obtenu à partir d'*Ophiopogon japonicus* selon l'invention. La résistance transépithéliale est mesurée et exprimée en $\Omega$ cm$^2$.

Les résultats sont présentés dans le Tableau 24 ci-dessous.

*Tableau 24*

| | Résistance transépithéliale ($\Omega$ cm$^2$) | Efficacité / Témoin REhBAI |
|---|---|---|
| REhBAI | | |
| Témoin | 317 | |
| Exemple contenant 0.075% du PA | 478 | 51% |
| Exemple contenant 0.15% du PA | 510 | 61% |

On constate que le principe actif obtenu à partir d'*Ophiopogon japonicus* permet d'augmenter la résistance transépithéliale dans le modèle REhBAI.

L'utilisation d'un principe actif obtenu à partir d'un hydrolysat d'*Ophiopogon japonicus* permet par conséquent, en application topique ou systémique, de protéger l'intégrité et la résistance de la barrière épidermique des peaux atopiques.

### 3/ *Effet sur le microbiote cutané*

En contact direct avec l'environnement, la peau est colonisée par un grand nombre de microorganismes : bactéries, levures, champignons, virus et acariens. Les connaissances actuelles établissent que la majorité de ces microorganismes sont inoffensifs pour l'homme. L'hôte et le microbiote vivent en symbiose (Salava et al., « Role of the skin microbiome in atopic dermatitis » Clinical and translational allergy, 4, 1-6, 2014).

Au cours des cinq dernières années, les recherches sur le microbiote ont connu un essor considérable. Cet essor s'explique par les importants progrès techniques réalisés sur les outils d'analyse bactérienne. La cartographie intégrale du microbiote cutané a ainsi pu être établie.

Bien qu'inoffensif pour l'individu, le microbiote n'est pas pour autant sans incidence pour la peau. En effet, en activant la réponse immune, les germes commensaux permettent à la peau de produire des peptides antimicrobiens (cathelicidines, β-defensines) qui vont prévenir l'invasion par des bactéries indésirables. Ces bactéries activent également la réponse Th1 au détriment de la réponse Th2, voie impliquée dans le développement de la dermatite atopique (Powers et al., « Microbiome and pediatric atopic dermatitis », Journal of Dermatology, 42, 1137-1142, 2015). Ces données décrivent à quel point le microbiote est important pour le maintien d'une peau saine.

Dans le cas de la dermatite atopique, le microbiote cutané est drastiquement modifié. Les zones de lésion présentent une diversité microbienne réduite au profit d'une prolifération du genre *Staphylococcus.* Deux types de staphylocoques sont décrits comme fortement présents : *Staphylococcus aureus* et *Staphylococcus epidermidis* dans une moindre mesure. 80 à 100% des lésions atopiques révèlent une présence de *Staphylococcus aureus* contre seulement 5 à 20% des zones saines. Cette colonisation anormale résulte d'un abaissement des défenses immunitaires qui accompagne cette pathologie et qui favorise l'implantation de bactéries indésirables. En retour, *Staphylococcus aureus* va accentuer les défauts de fonction barrière en sécrétant des superantigènes ou toxines qui vont stimuler la prolifération des lymphocytes T et réduire encore la production des peptides antimicrobiens (Williams et al., « The Role of the Skin Microbiome in Atopic Dermatitis, Curr Allergy Astham Rep, 15, 2-10, 2015, Thomas et al., « The microbiome and atopic eczema : more than skin deep », Australian journal of Dermatology, 2016).

La composante bactérienne de la peau constitue donc un paramètre prépondérant dans le développement de la dermatite atopique. A ce jour, certains traitements sont décrits comme capables de rééquilibrer l'écologie bactérienne au niveau des lésions. Dans le même sens, les thérapies améliorant le microbiote cutané des peaux atopiques sont associées à une amélioration du tableau clinique des patients (Flores et al., « Microbiome of affected and unaffected skin patients with atopic dermatitis before and after emolient treatment », Journal of drugs in dermatology, 13, 1365-1371, 2014 ; Seité et al., « Barrier function and microbiotic dysbiosis in atopic dermatitis », Clinical, cosmetic and investigational dermatology, 8, 479-483, 2015).

○ EVALUATION SUR LA FORMATION DU BIOFILM INDUITE PAR STAPHYLOCOCCUS AUREUS

L'effet du principe actif selon l'invention sur le microbiote cutané a été évalué *in vitro* sur des épidermes reconstruits humains après application d'une suspension de *Staphylococcus aureus.*

Les épidermes reconstruits humains normaux sont pré-traités topiquement pendant 24 heures avec le principe actif selon l'invention à 0,15% (V/V), puis traités avec une suspension de *S. aureus* ($10^6$ CFU/mL) pendant 24 heures.

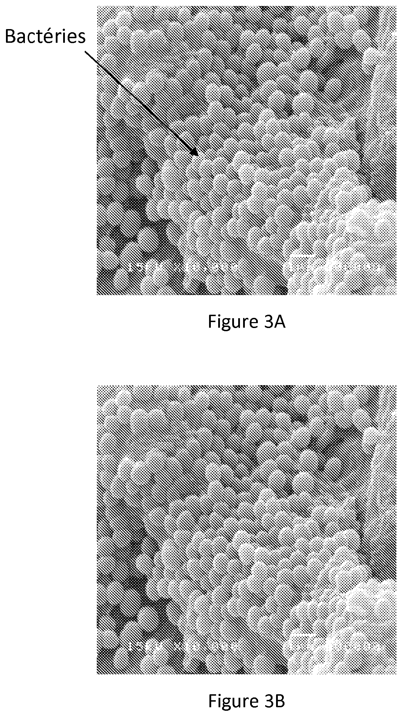

L'adhésion / colonisation par *Staphylococcus aureus* (biofilm) a été visualisée par microscopie électronique à balayage sur les figures 3A (Formation du biofilm induite par S. aureus sur le REh témoin) et 3B (formation du biofilm induite par S. aureus sur le REh traité par le principe actif selon l'invention).

*Tableau 25*

| | REh témoin | REh traité par le principe actif selon l'invention 0.15% |
|---|---|---|
| Formation du biofilm | +++ | + |

Appliqué en topique à 0,15% sur épidermes reconstruits humains normaux soumis à une agression bactérienne, le principe actif selon l'invention limite l'adhésion de *Staphylococcus aureus* et consécutivement la formation du biofilm.

**Revendications**

1. Principe actif obtenu à partir d'*Ophiopogon japonicus* pour une utilisation en application topique sur la peau dans le traitement de la dermatite atopique chez l'être humain ou chez l'animal, **caractérisé en ce qu'**il s'agit d'un hydrolysat d'*Ophiopogon japonicus.*

2. Principe actif pour son utilisation selon l'une des précédentes revendications sur des peaux atopiques humaines ou animales.

3. Principe actif pour une utilisation selon l'une des précédentes revendications, dans le traitement de la dermatite atopique du chien ou du chat.

**4.** Principe actif pour son utilisation selon l'une des précédentes revendications, pour une utilisation dans le traitement de la dermatite atopique en agissant sur :

- la fonction barrière cutanée, et/ou
- l'inflammation cutanée, et/ou
- le microbiote cutané.

**5.** Principe actif pour son utilisation selon l'une des précédentes revendications, pour une utilisation dans le traitement de la dermatite atopique en agissant :

- sur les marqueurs de l'inflammation des cellules, et/ou
- sur les gènes associés à l'inflammation des cellules cutanées, et/ou
- sur les marqueurs de la cohésion, et/ou
- sur les marqueurs de différenciation des cellules cutanées, et/ou
- sur les gènes associés à la différenciation des cellules cutanées, et/ou
- sur l'organisation et la conformation des lipides épidermiques, et/ou
- sur la construction morphologique de l'épiderme, et/ou
- sur l'intégrité et la résistance de la barrière épidermique, et/ou
- sur l'adhésion des bactéries sur la peau.

**6.** Principe actif pour son utilisation selon l'une des précédentes revendications, pour une utilisation dans le traitement des dermatites atopiques en agissant :

- en diminuant la teneur en TSLP ou en IL-8 dans les cellules cutanées, et/ou
- en normalisant l'expression du gène NELL2 et du gène Tenascine C dans les cellules cutanées, et/ou
- en augmentant la claudine-1 dans les cellules cutanées, et/ou
- en augmentant la filaggrine, la loricrine ou l'involucrine dans les cellules cutanées, et/ou
- en stimulant l'expression du gène filaggrine ou du gène loricrine dans les cellules cutanées, et/ou
- en diminuant l'expression du gène TGM1 (transglutaminase 1) dans les cellules cutanées, et/ou
- en limitant l'adhésion de *Staphylococcus aureus* sur la peau.

**7.** Principe actif pour son utilisation selon l'une des précédentes revendications, **caractérisé en ce qu'**il comprend des sucres.

**8.** Principe actif pour son utilisation selon l'une des précédentes revendications, **caractérisé en ce qu'**il comprend au moins 50% de sucre en poids par rapport au poids total de matière sèche.

**9.** Principe actif pour son utilisation selon l'une des revendications 7 ou 8, **caractérisé en ce qu'**il comprend des fructosanes.

**10.** Principe actif pour son utilisation selon la précédente revendication, **caractérisé en ce qu'**il comprend au moins 57% de fructosanes en poids par rapport au poids des sucres totaux du principe actif.

**11.** Principe actif pour son utilisation selon l'une des précédentes revendications, **caractérisé en ce qu'**il est obtenu à partir de tubercules d'Ophiopogon japonicus.

**12.** Principe actif pour son utilisation selon l'une des précédentes revendications, **caractérisé en ce qu'**il s'agit d'un hydrolysat enzymatique d'Ophiopogon japonicus.

**13.** Principe actif pour son utilisation selon l'une des précédentes revendications, dans une composition dermatologique à raison d'au moins 0.05%.

**14.** Principe actif pour son utilisation selon l'une des précédentes revendications, dans une composition dermatologique se présentant sous forme de crème, de gel, de lotion, de shampoing ou de pommade.

**Patentansprüche**

1. Wirkstoff, der aus *Ophiopogon japonicus* gewonnen wird, für eine Verwendung bei einer topischen Anwendung auf der Haut bei der Behandlung von atopischer Dermatitis bei einem Menschen oder bei einem Tier, **dadurch gekennzeichnet, dass** es sich um ein Hydrolysat von *Ophiopogon japonicus* handelt.

2. Wirkstoff für seine Verwendung nach einem der vorstehenden Ansprüche auf atopischen Häuten von Menschen oder Tieren.

3. Wirkstoff für seine Verwendung nach einem der vorstehenden Ansprüche bei der Behandlung von atopischer Dermatitis bei einem Hund oder einer Katze.

4. Wirkstoff für seine Verwendung nach einem der vorstehenden Ansprüche für eine Verwendung bei der Behandlung von atopischer Dermatitis durch Einwirkung auf:

   - die Hautbarrierefunktion, und/oder
   - die Hautentzündung, und/oder
   - die Hautflora.

5. Wirkstoff für seine Verwendung nach einem der vorstehenden Ansprüche für eine Verwendung bei der Behandlung von atopischer Dermatitis durch Einwirkung:

   - auf Zellentzündungsmarker, und/oder
   - auf Gene, die mit der Hautzellenentzündung verknüpft sind, und/oder
   - auf Kohäsionsmarker, und/oder
   - auf Hautzelldifferenzierungsmarker, und/oder
   - auf Gene, die mit der Differenzierung von Hautzellen verknüpft sind, und/oder
   - auf die Organisation und die Konformation epidermaler Lipide, und/oder
   - auf den morphologischen Aufbau der Epidermis, und/oder
   - auf die Integrität und die Widerstandsfähigkeit der epidermalen Barriere, und/oder
   - auf die Haftung von Bakterien an der Haut.

6. Wirkstoff für seine Verwendung nach einem der vorstehenden Ansprüche für eine Verwendung bei der Behandlung von atopischer Dermatitis durch Einwirkung:

   - durch Verringern des TSLP- oder IL-8-Gehalts in den Hautzellen, und/oder
   - durch Normalisieren der Expression des NELL2-Gens und des Tenascin C-Gens in den Hautzellen, und/oder
   - durch Erhöhen von Claudin-1 in Hautzellen, und/oder
   - durch Erhöhen von Filaggrin, Loricrin oder Involucrin in den Hautzellen, und/oder
   - durch Stimulieren der Expression des Filaggrin-Gens oder des Loricrin-Gens in den Hautzellen, und/oder
   - durch Verringern der Expression des TGM1-Gens (Transglutaminase 1) in den Hautzellen, und/oder
   - durch Begrenzen der Haftung von *Staphylococcus aureus* an der Haut.

7. Wirkstoff für seine Verwendung nach einem der vorstehenden Ansprüche, **dadurch gekennzeichnet, dass** er Zucker umfasst.

8. Wirkstoff für seine Verwendung nach einem der vorstehenden Ansprüche, **dadurch gekennzeichnet, dass** er zu mindestens 50 Gewichtsprozent Zucker umfasst, bezogen auf das Gesamtgewicht der Trockenmasse.

9. Wirkstoff für seine Verwendung nach einem der Ansprüche 7 oder 8, **dadurch gekennzeichnet, dass** er Fructosane umfasst.

10. Wirkstoff für seine Verwendung nach dem vorstehenden Anspruch, **dadurch gekennzeichnet, dass** er zu mindestens 57 Gewichtsprozent Fructosane umfasst, bezogen auf das Gewicht der Gesamtzucker des Wirkstoffs.

11. Wirkstoff für seine Verwendung nach einem der vorstehenden Ansprüche, **dadurch gekennzeichnet, dass** er aus Knollen von Ophiopogon japonicus gewonnen wird.

12. Wirkstoff für seine Verwendung nach einem der vorstehenden Ansprüche, **dadurch gekennzeichnet, dass** es sich um ein enzymatisches Hydrolysat von Ophiopogon japonicus handelt.

13. Wirkstoff für seine Verwendung nach einem der vorstehenden Ansprüche in einer dermatologischen Zusammensetzung in einer Menge von mindestens 0,05 %.

14. Wirkstoff für seine Verwendung nach einem der vorstehenden Ansprüche in einer dermatologischen Zusammensetzung in Form einer Creme, eines Gels, einer Lotion, eines Shampoos oder einer Salbe.

**Claims**

1. Active ingredient obtained from *Ophiopogon japonicus* for use in topical application to the skin in the treatment of atopic dermatitis in humans or animals, **characterized in that** it is a hydrolysate of *Ophiopogon japonicus.*

2. Active ingredient for use according to one of the preceding claims on atopic human or animal skin.

3. Active ingredient for use according to one of the preceding claims, in the treatment of atopic dermatitis in dogs or cats.

4. Active ingredient for use according to one of the preceding claims, for use in the treatment of atopic dermatitis by acting on:

   - skin barrier function, and/or
   - skin inflammation, and/or
   - skin microbiota.

5. Active ingredient for use according to one of the preceding claims, for use in the treatment of atopic dermatitis by acting:

   - on the cell inflammation markers, and/or
   - on the genes associated with skin cell inflammation, and/or
   - on the cohesion markers, and/or
   - on the skin cell differentiation markers, and/or
   - on the genes associated with skin cell differentiation, and/or
   - on the organization and conformation of epidermal lipids, and/or
   - on the morphological construction of the epidermis, and/or
   - on the integrity and resistance of the epidermal barrier, and/or
   - on the adhesion of bacteria to the skin.

6. Active ingredient for use according to one of the preceding claims, for use in the treatment of atopic dermatitis by acting:

   - by reducing the TSLP or IL-8 content in skin cells, and/or
   - by normalizing the expression of the NELL2 gene and Tenascin C gene in skin cells, and/or
   - by increasing claudin-1 in skin cells, and/or
   - by increasing filaggrin, loricrin or involucrin in skin cells, and/or
   - by stimulating the expression of the filaggrin gene or loricrin gene in skin cells, and/or
   - by reducing the expression of the TGM1 (transglutaminase 1) gene in skin cells, and/or
   - by limiting the adhesion of *Staphylococcus aureus* to the skin.

7. Active ingredient for use according to one of the preceding claims, **characterized in that** it comprises sugars.

8. Active ingredient for use according to one of the preceding claims, **characterized in that** it comprises at least 50% sugar by weight relative to the total weight of dry matter.

9. Active ingredient for use according to one of claims 7 or 8, **characterized in that** it comprises fructosans.

10. Active ingredient for use according to the preceding claim, **characterized in that** it comprises at least 57% fructosans by weight relative to the weight of the total sugars of the active ingredient.

11. Active ingredient for use according to one of the preceding claims, **characterized in that** it is obtained from Ophiopogon japonicus tubers.

12. Active ingredient for use according to one of the preceding claims, **characterized in that** it is an enzymatic hydrolysate of Ophiopogon japonicus.

13. Active ingredient for use according to one of the preceding claims, in a dermatological composition in an amount of at least 0.05%.

14. Active ingredient for use according to one of the preceding claims, in a dermatological composition in the form of cream, gel, lotion, shampoo or ointment.

Figure 1A

Figure 1B

Figure 1C

...

Figure 1D

Figure 1E

Figure 2A

Figure 2B

Figure 2C

Bactéries

Figure 3A

Figure 3B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014081715 A
- CN 103656323
- CN 103520572
- CN 102406570
- CN 101904985
- CN 103520475

- KR 20050014947
- WO 0247704 A
- CN 103736068
- CN 101757419, Ando Y et al., Toshiaki Makino
- FR 2930729

**Littérature non-brevet citée dans la description**

- **MARSELLA ; GIROLOMONI**. Canine models of atopic dermatitis: a useful tool with untapped potential. *J Invest Dermatol*, October 2009, vol. 129 (10), 2351-7
- **MARSELLA et al.** Current evidence of skin barrier dysfunction in human and canine atopic dermatitis. *Vetirinary Dermatology*, 2011, vol. 22, 239-248
- **BIZIKOWA et al.** Review : Clinical and histological manifestations of canine atopic dermatitis. *Vet Dermatol*, 2015, vol. 26, 79-e24
- **BIZIKOWA et al.** Review : Role of genetics and the environement in the pathogenesis of canine atopic dermatitis. *Vet Dermatol*, 2015, vol. 26, 95-e26
- **OLIVRY et al.** Early Activation of Th2/Th22 Inflammatory and Pruritogenic Pathways in Acute Canine Atopic Dermatitis Skin Lesions. *Journal of Investicative Dermatology*, 2016
- **P. ROUAUD-TINGUELY et al.** From the morphological to the transcriptomic characterization of a compromised three-dimensional in vitro model mimicking atopic dermatitis. *British Journal of Dermatology*, 2015, vol. 173, 1006-1014
- **DUBOIS M et al.** *Analytical chemistry*, 1956, vol. 28 (3), 350-356
- *British Journal of Dermatology*, 2015, vol. 173, 1006-1014
- **SKABYTSKA et al.** The role of innate immune signaling in the pathogenesis of atopic dermatitis and consequences for treatments. *Semin Immunopathol*, 2016, vol. 38, 29-43
- **AGRAWAL et al.** Skin barrier defects in atopic dermatitis. *Current allergy Asthma Rep*, 2014, vol. 14, 1-11
- **AMANAKA**. The role of cytokines/chemokines in the pathogenesis of atopic dermatitis. *Curr Probl Dermatol.*, 2011, vol. 41, 80-92

- **STALDER**. Fragility of epidermis and its consequence in dermatology. *J Eur Acad Dermatol Venereol.*, June 2014, vol. 28 (4), 1-18
- **KLUKOWSKA-ROTZLER**. Expression of thymic stromal lymphopoietin in canine atopic dermatitis. *Vet Dermatol*, February 2013, vol. 24 (1), 54-9.e13, 4
- **KAMSTEEG**. Type 2 Helper T-Cell Cytokines induce morphologic and Molecular characteristics of Atopic Dermatitis in Human Skin Equivalent. *Journal of investigative dematology*, 2007, vol. 127, 1786-1789
- **AGRAWAL ; FEINGOLD et al.** Role of lipids in the formation and maintenance of the cutaneous permeability barrier. *Biochim Biophys Act mars*, 2014, vol. 1841 (3), 280-94
- **SUGIURA et al.** Large-scale DNA microarray analytsis of atopic lesions shows of on epidermis differentiation gene cluster in the alternative pathway and lack of protective gene expression in the cornified envelope. *BJD*, January 2015, vol. 152 (Issue1), 146-149
- **ZHANG**. Screening for key genes associated with atopic dermatitis with DNA microarrays. *Molecular Medecine Reports*, March 2014, vol. 9 (3), 1049-1055
- **ELIAS et al.** Lipid abnormalities and lipid-based repair strategies in atopic dermatitis. *Biochimics et Biophyiscs acta*, 2014, vol. 1841, 323-330
- **VAN SMEDEN et al.** The important role of stratum corneum lipids for the cutaneous barrier function. *Biochemica et biophysica acta*, 2014, vol. 1841, 295-313
- **SALAVA et al.** Role of the skin microbiome in atopic dermatitis. *Clinical and translational allergy*, 2014, vol. 4, 1-6
- **POWERS et al.** Microbiome and pediatric atopic dermatitis. *Journal of Dermatology*, 2015, vol. 42, 1137-1142

- **WILLIAMS et al.** The Role of the Skin Microbiome in Atopic Dermatitis. *Curr Allergy Astham Rep*, 2015, vol. 15, 2-10
- **THOMAS et al.** The microbiome and atopic eczema : more than skin deep. *Australian journal of Dermatology*, 2016
- **FLORES et al.** Microbiome of affected and unaffected skin patients with atopic dermatitis before and after emolient treatment. *Journal of drugs in dermatology*, 2014, vol. 13, 1365-1371
- **SEITÉ et al.** Barrier function and microbiotic dysbiosis in atopic dermatitis. *Clinical, cosmetic and investigational dermatology*, 2015, vol. 8, 479-483